# EUROPEAN PATENT SPECIFICATION

(11) **EP 3 324 968 B1**
(45) Date of publication and mention of the grant of the patent: **27.09.2023**
(21) Application number: 16827318.3
(22) Date of filing: 21.07.2016
(51) Int. Cl.: A61K 31/475, A61K 31/337, A61P 35/00, A61K 31/4725, A61P 35/02

(54) **THERAPEUTIC COMBINATIONS OF ORALLY ADMINISTERED PACLITAXEL AND A P-GP INHIBITOR FOR THE TREATMENT OF CANCER**
THERAPEUTISCHE KOMBINATIONEN AUS ORAL VERABREICHTEM PACLITAXEL UND EINEM P-GP-HEMMER ZUR BEHANDLUNG VON KREBS
COMBINAISONS THÉRAPEUTIQUES DE PACLITAXEL ET D'UN INHIBITEUR DE P-GP À USAGE ORAL POUR LE TRAITEMENT DU CANCER

(30) Priority: 21.07.2015 US 201562195243 P
(43) Date of publication of application: 30.05.2018
(73) Proprietor: Athenex Therapeutics Limited, Hong Kong (CN)
(72) Inventor: KWAN, Min-fun, Rudolf, Summit, NJ 07901 (US); KRAMER, E., Douglas, Stamford, CT 06905 (US); FETTERLY, Geralk, J., Getezville, NY 14068 (US); HUNG, Cheung-tak, Dunedin 9010 (NZ); JACKSON, Christopher Glyn Charles, Alexander, Dunedin 9012 (NZ); GLUE, Paul, William, Dunedin 9054 (NZ)
(74) Representative: Dolphin, Kirsty Mairi
(86) International application number: PCT/IB2016/001132
(87) International publication number: WO 2017/013490

(56) References cited:
- HYUN JUNG LEE ET AL: "A Phase I Study of Oral Paclitaxel with a Novel P-Glycoprotein Inhibitor, HM30181A, in Patients with Advanced Solid Cancer", CANCER RESEARCH AND TREATMENT, vol. 46, no. 3, 1 July 2014 (2014-07-01), pages 234-242, XP055561439, KR ISSN: 1598-2998, DOI: 10.4143/crt.2014.46.3.234
- SEBASTIAN C.KO¨HLER ET AL.: 'HM30181 Derivatives as Novel Potent and Selective Inhibitors of the Breast Cancer Resistance Protein (BCRP/ABCG2' JOURNAL OF MEDICINAL CHEMISTRY vol. 58, 09 April 2015, pages 3910 - 3921, XP055365386
- JIN-OH KWAK ET AL.: 'Selective inhibition of MDR1 (ABCB1) by HM30181 increases oral bioavailability and therapeutic efficacy of paclitaxel' EUROPEAN JOURNAL OF PHARMACOLOGY vol. 627, 31 December 2010, pages 92 - 98, XP026832548

## Description

### RELATED APPLICATION

This application claims priority to, and the benefit of, U.S. Provisional Application No. 62/195,243, filed July 21, 2015.

### BACKGROUND

Paclitaxel (Taxol^{®}) was co-developed by the U.S. National Cancer Institute and Bristol-Myers Squibb, and was approved by the U.S. Food and Drug Administration as an anti-cancer drug in 1992, and marketed under the brand name Taxol^{®}. Paclitaxel stabilizes microtubules in the cell, thereby interfering with the normal breakdown of microtubules during mitosis. Due to this unique mechanism which restrains cancer cell division without detrimental effects on DNA synthesis, Paclitaxel is indicated to treat many types of cancer, including lung, ovarian and breast.

However, the affinity of paclitaxel for the p-glycoprotein pump (P-gp) leads to efflux of paclitaxel back into the intestinal lumen, thereby making the drug non-bioavailable when taken orally. Its poor absorption through the intestinal epithelium, along with its unfavorable solubility, has necessitated intravenous administration of paclitaxel. Excipients, such as Cremophor^{®}, used for intravenous administration of paclitaxel often cause tolerability problems such as hypersensitivity-type infusion reactions. Intravenously administered paclitaxel (e.g., Taxol^{®} or paclitaxel formulated with Cremophor^{®}) requires premedication which has its own set of side effects. Further, intravenously administered paclitaxel may also be associated with increased incidence or severity of neurotoxicity.

Therefore, an effective therapeutic regimen including an oral formulation of paclitaxel along with oral administration of a P-gp inhibitor may be beneficial and may be expected to improve the treatment outcomes of cancer. A phase I study of oral paclitaxel in a P-gp inhibitor in patients with advanced solid cancer is described in Hyun Jung Lee et al. Cancer Res Treat. 2014;46(3):234-242. By inhibiting the efflux of paclitaxel by P-gp back to the intestinal lumen, oral administration would allow therapeutically relevant concentrations of the drug that are now efficacious and also would avoid the use of excipients such as polyethoxylated castor oil, *e.g.,* Cremophor^{®}, thus leading to a wide therapeutic window that will promote antitumor response while mitigating or avoiding the reactions and toxicities associated directly with the drug or the excipients. In addition, oral administration of paclitaxel provides a more convenient and safe method. The present application addresses the needs for orally administering paclitaxel.

### SUMMARY

The application pertains to a compound for use in the treatment of cancer in a subject in need thereof, wherein the subject is administered paclitaxel orally at an amount of about 100 mg/m² to about 250 mg/m² once a day and for 1-7 times a week;
wherein the compound is Compound A: 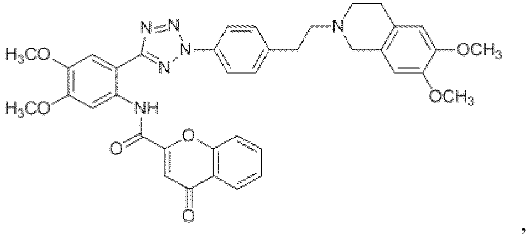 which is administered` to the subject at an amount of about 15 mg, about 20 mg, about 25 mg, or about 30 mg once a day and for 1-7 times a week; and
wherein Compound A is administered simultaneously with or prior to the paclitaxel.

The application pertains to a compound for use in reducing hematologic toxicity and/or neurotoxicity in a subject suffering from cancer and undergoing paclitaxel therapy, wherein the subject is administered paclitaxel orally at an amount of about 100 mg/m² to about 250 mg/m² once a day and for 1-7 times a week;
wherein the compound is Compound A: 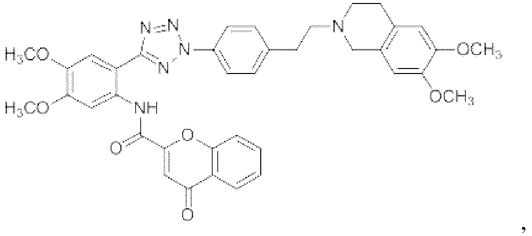 which is administered to the subject at an amount of about 15 mg, about 20 mg, about 25 mg, or about 30 mg once a day and for 1-7 times a week; and
wherein the plasma exposure of the orally administered paclitaxel, as measured by AUC_{(0→∞)}, is equal to or greater than the plasma exposure, as measured by AUC_{(0→∞)}, of intravenously administered paclitaxel (e.g., Taxol^{®} or paclitaxel formulated with Cremophor@) at an amount of about 135 mg/m² to 175 mg/m² over a period of about 3 to about 24 hours once every 3 weeks, and wherein Compound A is administered simultaneously with or prior to the paclitaxel.

The application pertains to a compound for use in reducing or preventing hypersensitivity-type infusion reactions associated with intravenously administered paclitaxel (e.g., Taxol^{®} or paclitaxel formulated with Cremophor@) therapy in a subject suffering from cancer, wherein the subject is administered paclitaxel orally at an amount of about 100 mg/m² to about 250 mg/m² once a day and for 1-7 times a week;
wherein the compound is Compound A: 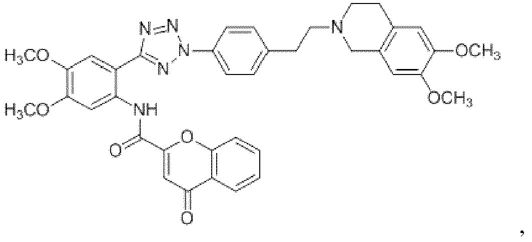 which is administered to the subject at an amount of about 15 mg, about 20 mg, about 25 mg, or about 30 mg once a day and for 1-7 times a week; and
wherein the orally administered paclitaxel reaches therapeutic blood or plasma levels in the subject, and
wherein Compound A is administered simultaneously with or prior to the paclitaxel.

### DETAILED DESCRIPTION

The description pertains, at least in part, to methods for treating cancer in a subject.

The description pertains, at least in part, to methods for reducing or preventing toxicity, hypersensitivity-type infusion reactions, and other negative outcomes resulting from or associated with intravenously administered paclitaxel (*e.*g., Taxol^{®} or paclitaxel formulated with Cremophor@) therapy in a subject suffering from cancer.

In one example, the application pertains to a method for treating cancer in a subject in need thereof, comprising:
a. oral administration of paclitaxel at an amount of about 100 mg/m² to about 400 mg/m² to the subject once a day and for 1-7 times a week; and
b. oral administration of Compound A: 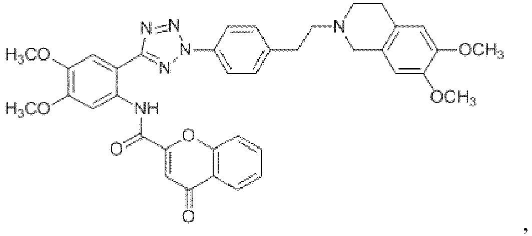 to the subject once a day and for 1-7 times a week, wherein Compound A is administered simultaneously with or prior to the paclitaxel.

In one example, the application pertains to a method for reducing hematologic toxicity and/or neurotoxicity in a subject suffering from cancer and undergoing a paclitaxel therapy, comprising:
a. oral administration of paclitaxel at an amount of about 100 mg/m² to about 400 mg/m² to the subject once a day and for 1-7 times a week; and
b. oral administration of Compound A: 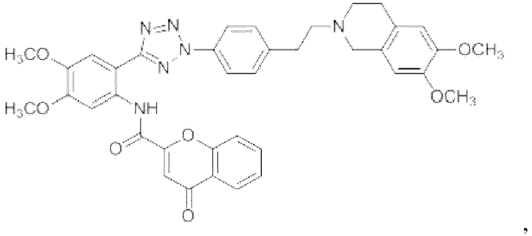 to the subject once a day and for 1-7 times a week, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor^{®}) at an amount of about 135 mg/m² to 175 mg/m² over a period of about 3 hours to about 24 hours once every 3 weeks, and wherein Compound A is administered simultaneously with or prior to the paclitaxel.

The description pertains, at least in part, to a compound for use in the treatment of cancer in a subject.

The description pertains, at least in part, to a compound for use in reducing or preventing toxicity, hypersensitivity-type infusion reactions, and other negative outcomes resulting from or associated with intravenously administered paclitaxel (*e.g.,* Taxol^{®} or paclitaxel formulated with Cremophor@) therapy in a subject suffering from cancer.

In one example, the application pertains to a compound for use in the treatment of cancer in a subject in need thereof, wherein the subject is administered paclitaxel orally at an amount of about 100 mg/m² to about 400 mg/m² once a day and for 1-7 times a week;
wherein the compound is Compound A: 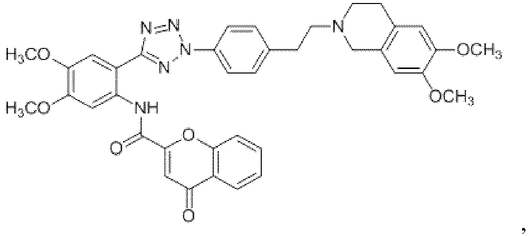 which is administered to the subject once a day and for 1-7 times a week; and
wherein Compound A is administered simultaneously with or prior to the paclitaxel.

In one example, the application pertains to a compound for use in reducing hematologic toxicity and/or neurotoxicity in a subject suffering from cancer and undergoing paclitaxel therapy, wherein the subject is administered paclitaxel orally at an amount of about 100 mg/m² to about 400 mg/m² once a day and for 1-7 times a week;
wherein the compound is Compound A: 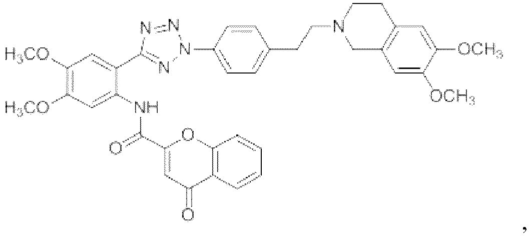 , which is administered to the subject once a day and for 1-7 times a week; and
wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² to 175 mg/m² over a period of about 3 to about 24 hours once every 3 weeks, and wherein Compound A is administered simultaneously with or prior to the paclitaxel.

The description pertains, at least in part, to the use of a compound in the manufacture of a medicament for the treatment of cancer in a subject.

The description pertains, at least in part, to the use of a compound in the manufacture of a medicament for use in reducing or preventing toxicity, hypersensitivity-type infusion reactions, and other negative outcomes resulting from or associated with intravenously administered paclitaxel (*e.g.,* Taxol^{®} or paclitaxel formulated with Cremophor@) therapy in a subject suffering from cancer.

In one example, the description pertains to the use of a compound in the manufacture of a medicament for treating cancer in a subject in need thereof, wherein the subject is administered paclitaxel orally at an amount of about 100 mg/m² to about 400 mg/m² once a day and for 1-7 times a week;
wherein the compound is Compound A: 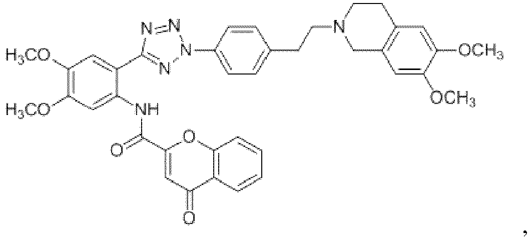 which is administered to the subject once a day and for 1-7 times a week; and
wherein Compound A is administered simultaneously with or prior to the paclitaxel.

In one example, the description pertains to the use of a compound in the manufacture of a medicament for use in reducing hematologic toxicity and/or neurotoxicity in a subject suffering from cancer and undergoing paclitaxel therapy, wherein the subject is administered paclitaxel orally at an amount of about 100 mg/m² to about 400 mg/m² once a day and for 1-7 times a week;
wherein the compound is Compound A: 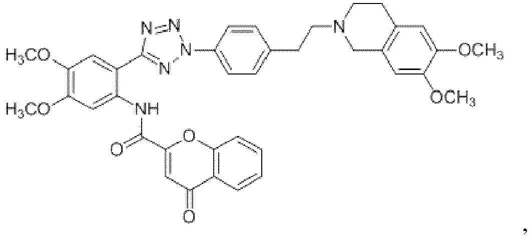 which is administered to the subject once a day and for 1-7 times a week; and
wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor^{®}) at an amount of about 135 mg/m² to 175 mg/m² over a period of about 3 to about 24 hours once every 3 weeks, and
wherein Compound A is administered simultaneously with or prior to the paclitaxel.

The description pertains, at least in part, to a compound for use in a combination therapy in the treatment of cancer in a subject.

The description pertains, at least in part, to a compound for use in a combination therapy for use in reducing or preventing toxicity, hypersensitivity-type infusion reactions, and other negative outcomes resulting from or associated with intravenously administered paclitaxel (*e.g*., Taxol^{®} or paclitaxel formulated with Cremophor@) therapy in a subject suffering from cancer.

In one example, the description pertains to a compound for use in a combination therapy in the treatment of cancer in a subject in need thereof, wherein the compound is Compound A: 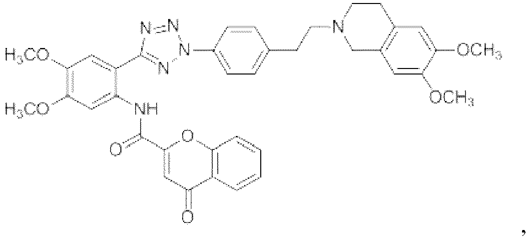 which is administered to the subject once a day and for 1-7 times a week;
wherein the subject is also administered paclitaxel orally at an amount of about 100 mg/m² to about 400 mg/m² once a day and for 1-7 times a week; and
wherein Compound A is administered simultaneously with or prior to the paclitaxel.

In one example, the description pertains to a compound for use in a combination therapy for reducing hematologic toxicity and/or neurotoxicity in a subject suffering from cancer and undergoing paclitaxel therapy, wherein the compound is Compound A: 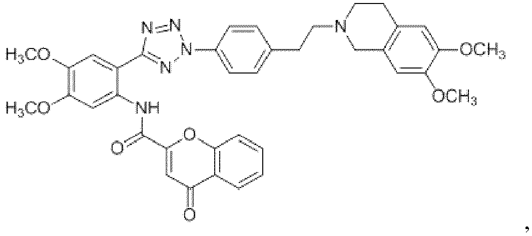 which is administered to the subject once a day and for 1-7 times a week;
wherein the subject is also administered paclitaxel orally at an amount of about 100 mg/m² to about 400 mg/m² once a day and for 1-7 times a week;
wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to 175 mg/m² over a period of about 3 to about 24 hours once every 3 weeks; and
wherein Compound A is administered simultaneously with or prior to the paclitaxel.

The description pertains, at least in part, to a medicament for use in a combination therapy in the treatment of cancer in a subject.

The description pertains, at least in part, to a medicament for use in a combination therapy for use in reducing or preventing toxicity, hypersensitivity-type infusion reactions, and other negative outcomes resulting from or associated with intravenously administered paclitaxel (*e.g.*, Taxol^{®} or paclitaxel formulated with Cremophor@) therapy in a subject suffering from cancer.

In one example, the description pertains to a medicament for use in a combination therapy in the treatment of cancer in a subject in need thereof, wherein the medicament comprises Compound A: 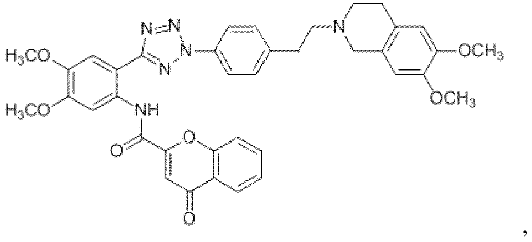 which is administered to the subject once a day and for 1-7 times a week;
wherein the subject is also administered paclitaxel orally at an amount of about 100 mg/m² to about 400 mg/m² once a day and for 1-7 times a week; and
wherein the medicament is administered simultaneously with or prior to the paclitaxel.

In one example, the description pertains to a medicament for use in a combination therapy for reducing hematologic toxicity and/or neurotoxicity in a subject suffering from cancer and undergoing paclitaxel therapy, wherein the medicament comprises Compound A: 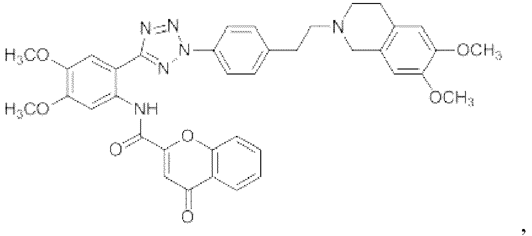 which is administered to the subject once a day and for 1-7 times a week;
wherein the subject is also administered paclitaxel orally at an amount of about 100 mg/m² to about 400 mg/m² once a day and for 1-7 times a week;
wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to 175 mg/m² over a period of about 3 to about 24 hours once every 3 weeks; and
wherein the medicament is administered simultaneously with or prior to the paclitaxel.

In one embodiment, the intravenously administered paclitaxel (*e.g*., Taxol^{®} or paclitaxel formulated with Cremophor^{®}) is infused over a period of about 3 hours once every 3 weeks.

In one embodiment, the intravenously administered paclitaxel (*e.g*., Taxol^{®} or paclitaxel formulated with Cremophor^{®}) is infused over a period of about 6 hours once every 3 weeks.

In one embodiment, the intravenously administered paclitaxel (*e.g*., Taxol^{®} or paclitaxel formulated with Cremophor^{®}) is infused over a period of about 9 hours once every 3 weeks.

In one embodiment, the intravenously administered paclitaxel (*e.g*., Taxol^{®} or paclitaxel formulated with Cremophor^{®}) is infused over a period of about 12 hours once every 3 weeks.

In one embodiment, the intravenously administered paclitaxel (*e.g*., Taxol^{®} or paclitaxel formulated with Cremophor^{®}) is infused over a period of about 15 hours once every 3 weeks.

In one embodiment, the intravenously administered paclitaxel (*e.g*., Taxol^{®} or paclitaxel formulated with Cremophor^{®}) is infused over a period of about 18 hours once every 3 weeks.

In one embodiment, the intravenously administered paclitaxel (*e.g*., Taxol^{®} or paclitaxel formulated with Cremophor^{®}) is infused over a period of about 21 hours once every 3 weeks.

In one embodiment, the intravenously administered paclitaxel (*e.g*., Taxol^{®} or paclitaxel formulated with Cremophor^{®}) is infused over a period of about 24 hours once every 3 weeks.

In one embodiment, the hematologic toxicity associated with the intravenous administration of paclitaxel in a subject suffering from cancer includes anemia, and myelosuppression. In one embodiment, the myelosuppression may be from leukopenia, neutropenia, thrombocytopenia, or any combination thereof.

In one embodiment, the neurotoxicity associated with the intravenous administration of paclitaxel in a subject suffering from cancer includes symptoms such as numbness, tingling, sharp pain, jabbing pain, burning pain, extreme sensitivity, loss of coordination, falling, weakness, paralysis, sweating, heat intolerance, dizziness, changes in blood pressure, bowel problems, bladder problems, or any combination thereof.

In one example, the description pertains to a method for reducing or preventing hypersensitivity-type infusion reactions associated with intravenously administered paclitaxel *(e.g.,* Taxol^{®} or paclitaxel formulated with Cremophor@) therapy in a subject suffering from cancer, comprising:
a. oral administration of paclitaxel at an amount of about 100 mg/m² to about 400 mg/m² to the subject once a day and for 1-7 times a week; and
b. oral administration of Compound A: 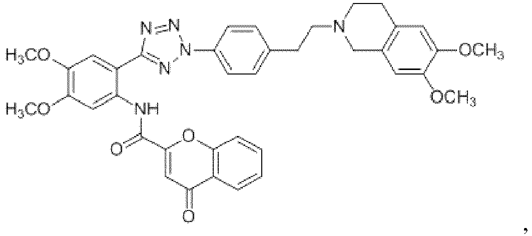 to the subject once a day and for 1-7 times a week, wherein the orally administered paclitaxel reaches therapeutic blood or plasma levels in the subject, and
   wherein Compound A is administered simultaneously with or prior to the paclitaxel.

In one example, the description pertains to a compound for use in reducing or preventing hypersensitivity-type infusion reactions associated with intravenously administered paclitaxel (*e.g*., Taxol^{®} or paclitaxel formulated with Cremophor@) therapy in a subject suffering from cancer, wherein the subject is administered paclitaxel orally at an amount of about 100 mg/m² to about 400 mg/m² once a day and for 1-7 times a week;
wherein the compound is Compound A: 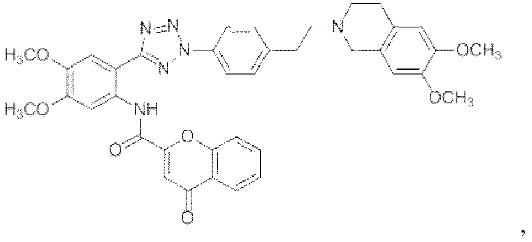 which is administered to the subject once a day and for 1-7 times a week; and
wherein the orally administered paclitaxel reaches therapeutic blood or plasma levels in the subject, and
wherein Compound A is administered simultaneously with or prior to the paclitaxel.

In one example, the description pertains to the use of a compound in the manufacture of a medicament for use in reducing or preventing hypersensitivity-type infusion reactions associated with intravenously administered paclitaxel (*e.g.*, Taxol^{®} or paclitaxel formulated with Cremophor@) therapy in a subject suffering from cancer, wherein the subject is administered paclitaxel orally at an amount of about 100 mg/m² to about 400 mg/m² once a day and for 1-7 times a week;
wherein the compound is Compound A: 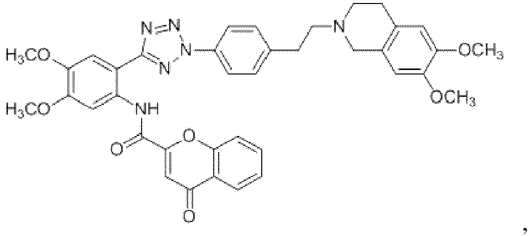 which is administered to the subject once a day and for 1-7 times a week; and
wherein the orally administered paclitaxel reaches therapeutic blood or plasma levels in the subject, and
wherein Compound A is administered simultaneously with or prior to the paclitaxel.

In one example, the description pertains to a compound for use in a combination therapy for reducing or preventing hypersensitivity-type infusion reactions associated with intravenously administered paclitaxel (*e.g*., Taxol^{®} or paclitaxel formulated with Cremophor@) therapy in a subject suffering from cancer wherein the compound is Compound A: 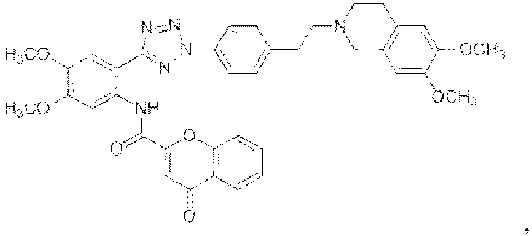 which is administered to the subject once a day and for 1-7 times a week; and
wherein the subject is also administered paclitaxel orally at an amount of about 100 mg/m² to about 400 mg/m² once a day and for 1-7 times a week; and wherein the orally administered paclitaxel reaches therapeutic blood or plasma levels in the subject, and
wherein Compound A is administered simultaneously with or prior to the paclitaxel.

In one example, the description pertains to a medicament for use in a combination therapy for reducing or preventing hypersensitivity-type infusion reactions associated with intravenously administered paclitaxel (*e.g*., Taxol^{®} or paclitaxel formulated with Cremophor@) therapy in a subject suffering from cancer wherein the medicament comprises Compound A: 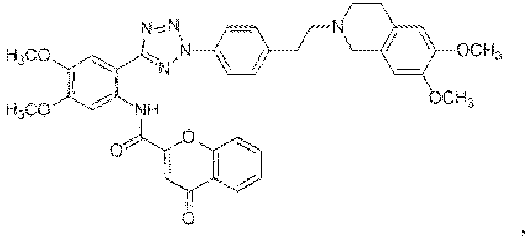 which is administered to the subject once a day and for 1-7 times a week; and
wherein the subject is also administered paclitaxel orally at an amount of about 100 mg/m² to about 400 mg/m² once a day and for 1-7 times a week; and wherein the orally administered paclitaxel reaches therapeutic blood or plasma levels in the subject, and wherein the medicament is administered simultaneously with or prior to the paclitaxel.

In one embodiment, the hypersensitivity-type infusion reactions associated with the intravenous administration of paclitaxel in a subject suffering from cancer includes any sign or symptom on the first day of intravenous administration of paclitaxel. In one embodiment, the signs or symptoms in the subject include fever, rash, hives, pruritus, flushing, swelling, dyspnea, bronchospasm, stridor, reduced pulmonary expiratory flow, hypoxia, hypertension, hypotension, hypotonia, syncope, falling, incontinence, abdominal pain, vomiting, urticaria, facial swelling, eye disorders, headache, arrhythmia, tachycardia, nausea, chest pain, anaphylaxis, or any combination thereof.

The above examples of the description include examples where the paclitaxel is administered orally at an amount of about 100 mg/m² to about 400 mg/m².

In some examples, the paclitaxel is administered at an amount of about 100 mg/m² to about 350 mg/m².

In some examples, the paclitaxel is administered at an amount of about 150 mg/m² to about 350 mg/m².

In some embodiments, the paclitaxel is administered at an amount of about 150 mg/m² to about 250 mg/m².

In some examples, the paclitaxel is administered at an amount of about 200 mg/m² to about 300 mg/m².

In some examples, the paclitaxel is administered at an amount of about 250 mg/m² to about 350 mg/m².

In some embodiments, the paclitaxel is administered at an amount of about 100 mg/m².

In some embodiments, the paclitaxel is administered at an amount of about 150 mg/m².

In some embodiments, the paclitaxel is administered at an amount of about 200 mg/m².

In some embodiments, the paclitaxel is administered at an amount of about 250 mg/m².

In some examples, the paclitaxel is administered at an amount of about 300 mg/m².

In some examples, the paclitaxel is administered at an amount of about 350 mg/m².

In some examples, the paclitaxel is administered at an amount of about 400 mg/m².

The embodiments of the application include examples where the paclitaxel is administered 1-7 times per week. In some embodiments, the paclitaxel is administered on consecutive days.

The embodiments of the application include examples where the paclitaxel is administered 2-7 times per week. In some embodiments, the paclitaxel is administered on consecutive days.

The embodiments of the application include examples where the paclitaxel is administered 2-6 times per week. In some embodiments, the paclitaxel is administered 2-5 times per week. In some embodiments, the paclitaxel is administered 2-4 times per week. In some embodiments, the paclitaxel is administered 2-3 times per week. In some embodiments, the paclitaxel is administered on consecutive days.

The embodiments of the application include examples where the paclitaxel is administered 3-6 times per week. In some embodiments, the paclitaxel is administered 3-5 times per week. In some embodiments, the paclitaxel is administered 3-4 times per week. In some embodiments, the paclitaxel is administered on consecutive days.

The embodiments of the application include examples where the paclitaxel is administered 4-6 times per week. In some embodiments, the paclitaxel is administered 4-5 times per week. In some embodiments, the paclitaxel is administered on consecutive days.

The embodiments of the application include examples where the paclitaxel is administered 5-6 times per week. In some embodiments, the paclitaxel is administered on consecutive days.

The embodiments of the application include examples where the paclitaxel is administered at least 2 times per week. In some embodiments, the paclitaxel is administered on consecutive days.

In some examples, the paclitaxel is administered at least 2 times per week at an amount of about 150 mg/m² to about 400 mg/m².

In some examples, the paclitaxel is administered at least 2 times per week at an amount of about 200 mg/m² to about 400 mg/m².

In some examples, the paclitaxel is administered at least 2 times per week at an amount of about 250 mg/m² to about 350 mg/m².

In some embodiments, the paclitaxel is administered at least 2 times per week at an amount of about 150 mg/m², about 175 mg/m², about 200 mg/m², about 225 mg/m², or about 250 mg/m². In some examples, the paclitaxel is administered at least 2 times per week at an amount of about 275 mg/m², about 300 mg/m², about 325 mg/m², about 350 mg/m², about 375 mg/m², or about 400 mg/m². In some examples, the paclitaxel is administered at least 2 times per week at an amount of about 250 mg/m², about 275 mg/m², about 300 mg/m², about 325 mg/m², or about 350 mg/m².

The embodiments of the application include examples where the paclitaxel is administered at least 3 times per week. In some embodiments, the paclitaxel is administered on consecutive days.

In some examples, the paclitaxel is administered at least 3 times per week at an amount of about 150 mg/m² to about 350 mg/m².

In some examples, the paclitaxel is administered at least 3 times per week at an amount of about 150 mg/m² to about 300 mg/m².

In some embodiments, the paclitaxel is administered at least 3 times per week at an amount of about 150 mg/m² to about 250 mg/m².

In some embodiments, the paclitaxel is administered at least 3 times per week at an amount of about 150 mg/m², about 175 mg/m², about 200 mg/m², about 225 mg/m², or about 250 mg/m². In some examples, the paclitaxel is administered at least 3 times per week at an amount of about 275 mg/m², about 300 mg/m², about 325 mg/m², or about 350 mg/m². In some embodiments, the paclitaxel is administered at least 3 times per week at an amount of about 150 mg/m², about 175 mg/m², about 200 mg/m², about 205 mg/m², about 210 mg/m², about 215 mg/m², about 220 mg/m², about 225 mg/m², or about 250 mg/m².

The embodiments of the application include examples where the paclitaxel is administered at least 4 times per week. In some embodiments, the paclitaxel is administered on consecutive days.

In some examples, the paclitaxel is administered at least 4 times per week at an amount of about 100 mg/m² to about 350 mg/m².

In some embodiments, the paclitaxel is administered at least 4 times per week at an amount of about 125 mg/m² to about 250 mg/m².

In some embodiments, the paclitaxel is administered at least 4 times per week at an amount of about 125 mg/m² to about 200 mg/m².

In some embodiments, the paclitaxel is administered at least 4 times per week at an amount of about 100 mg/m², about 125 mg/m², about 150 mg/m², about 175 mg/m², about 200 mg/m², about 225 mg/m², or about 250 mg/m². In some examples, the paclitaxel is administered at least 4 times per week at an amount of about 275 mg/m², about 300 mg/m², about 325 mg/m², or about 350 mg/m². In some embodiments, the paclitaxel is administered at least 4 times per week at an amount of about 125 mg/m², about 150 mg/m², about 175 mg/m², or about 200 mg/m².

The embodiments of the application include examples where the paclitaxel is administered at least 5 times per week. In some embodiments, the paclitaxel is administered on consecutive days.

In some examples, the paclitaxel is administered at least 5 times per week at an amount of about 100 mg/m² to about 350 mg/m².

In some embodiments, the paclitaxel is administered at least 5 times per week at an amount of about 100 mg/m² to about 250 mg/m².

In some embodiments, the paclitaxel is administered at least 5 times per week at an amount of about 100 mg/m² to about 200 mg/m².

In some embodiments, the paclitaxel is administered at least 5 times per week at an amount of about 100 mg/m², about 125 mg/m², about 150 mg/m², about 175 mg/m², about 200 mg/m², about 225 mg/m², or about 250 mg/m². In some examples, the paclitaxel is administered at least 5 times per week at an amount of about 275 mg/m², about 300 mg/m², about 325 mg/m², or about 350 mg/m². In some embodiments, the paclitaxel is administered at least 5 times per week at an amount of about 100 mg/m², about 125 mg/m², about 150 mg/m², about 175 mg/m², or about 200 mg/m².

The embodiments of the application include examples where the paclitaxel is administered at least 6 times per week. In some embodiments, the paclitaxel is administered on consecutive days.

In some examples, the paclitaxel is administered at least 6 times per week at an amount of about 100 mg/m² to about 300 mg/m².

In some embodiments, the paclitaxel is administered at least 6 times per week at an amount of about 100 mg/m² to about 200 mg/m².

In some embodiments, the paclitaxel is administered at least 6 times per week at an amount of about 100 mg/m² to about 150 mg/m².

In some embodiments, the paclitaxel is administered at least 6 times per week at an amount of about 100 mg/m², about 125 mg/m², about 150 mg/m², about 175 mg/m², about 200 mg/m², about 225 mg/m², or about 250 mg/m². In some examples, the paclitaxel is administered at least 6 times per week at an amount of about 275 mg/m², or about 300 mg/m². In some embodiments, the paclitaxel is administered at least 6 times per week at an amount of about 100 mg/m², about 125 mg/m², or about 150 mg/m².

The examples of the description include examples where Compound A is administered orally at an amount of about 1 mg to about 500 mg.

In some examples, Compound A is administered orally at about 1 mg to 400 mg.

In some examples, Compound A is administered orally at about 1 mg to 300 mg.

In some examples, Compound A is administered orally at about 5 mg to 200 mg.

In some examples, Compound A is administered orally at about 10 mg to 100 mg.

In some examples, Compound A is administered orally at about 15 mg to 50 mg.

In some embodiments, Compound A is administered orally at about 15 mg.

In some embodiments, Compound A is administered orally at about 20 mg

In some embodiments, Compound A is administered orally at about 25 mg.

In some embodiments, Compound A is administered orally at about 30 mg

In some examples, Compound A is administered orally at about 35 mg.

In some examples, Compound A is administered orally at about 45 mg.

In some examples, Compound A is administered orally at about 50 mg.

The embodiments of the application include examples where Compound A and the paclitaxel are administered on the same day.

The embodiments of the application include examples where Compound A is administered before the paclitaxel is administered.

In some embodiments, Compound A is administered about 5 minutes before the paclitaxel is administered.

In some embodiments, Compound A is administered about 10 minutes before the paclitaxel is administered.

In some embodiments, Compound A is administered about 15 minutes before the paclitaxel is administered.

In some embodiments, Compound A is administered about 30 minutes before the paclitaxel is administered.

In some embodiments, Compound A is administered about 45 minutes before the paclitaxel is administered.

In some embodiments, Compound A is administered about 60 minutes before the paclitaxel is administered.

In some embodiments, Compound A is administered about 2 hours before the paclitaxel is administered.

In some embodiments, Compound A is administered about 3 hours before the paclitaxel is administered.

In some embodiments, Compound A is administered about 4 hours before the paclitaxel is administered.

In some embodiments, Compound A is administered about 6 hours before the paclitaxel is administered.

In some embodiments, Compound A is administered about 8 hours before the paclitaxel is administered.

The embodiments of the application include examples where the plasma exposure of the orally administered paclitaxel, as measured by AUC_{(0→∞),} is equal to or greater than the plasma exposure of intravenously administered paclitaxel (e.g., Taxol^{®} or paclitaxel formulated with Cremophor@) at an amount of about 80 mg/m² over a period of about 60 minutes, as measured by AUC_{(0→∞)}.

In some embodiments, the AUC_{(0→∞)} of the orally administered paclitaxel is equal to the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is greater than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is about 10, 15, 20, 25, 30, 35, 40, 45, 50, 55, 60, 65, 70, 75, 80, 85, 90, 95, or 100% greater than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 10% greater (*e.g.,* at least 10% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 20% greater (*e.g.,* at least 20% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 30% greater (*e.g.,* at least 30% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 40% greater (*e.g.,* at least 40% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 50% greater *(e.g.,* at least 50% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 60% greater (*e.g.,* at least 60% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 70% greater (*e.g.,* at least 70% greater) than the AUC_{(0→∞)} of intravenously administered paclitaxel (*e.g.*, Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 80% greater *(e.g.,* at least 80% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 90% greater *(e.g.,* at least 90% greater) than the AUC_{(0→∞)} of intravenously administered paclitaxel (*e.g*., Taxol^{®} or paclitaxel formulated with Cremophor@) at an amount of about 80 mg/m² over a period of about 60 minutes.

In some embodiments, the AUC_{(0→∞)} of the orally administered paclitaxel is at least about 100% greater *(e.g.,* at least 100% greater) than the AUC_{(0→∞)} of intravenously administered paclitaxel (*e.g.*, Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 100% greater than the AUC_{(0→∞)} of intravenously administered paclitaxel (*e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 90% greater than the AUC_{(0→∞)} of intravenously administered paclitaxel (*e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 80% greater than the AUC_{(0→∞)} of intravenously administered paclitaxel (*e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 70% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 60% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 50% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 40% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 35% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 30% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 25% greater than the AUC_{(0→∞)} of intravenously administered paclitaxel *(e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 10% greater to about 20% greater than the AUC_{(0→∞)} of intravenously administered paclitaxel (*e.g.,* Taxol^{®} or paclitaxel formulated with Cremophor@) at an amount of about 80 mg/m² over a period of about 60 minutes.

In some embodiments, the AUC_{(0→∞)} of the orally administered paclitaxel is about 15% greater to about 25% greater than the AUC_{(0→∞)} of intravenously administered paclitaxel (*e.g.,* Taxol^{®} or paclitaxel formulated with Cremophor@) at an amount of about 80 mg/m² over a period of about 60 minutes.

The methods of the application include embodiments where the AUC_{(0→∞)} of the orally administered paclitaxel is about 2,000 ng•h/mL to about 10,000 ng•h/mL.

In some embodiments, the AUC_{(0→∞)} of the orally administered paclitaxel is about 2,000 ng•h/mL to about 9,000 ng•h/mL.

In some embodiments, the AUC_{(0→∞)} of the orally administered paclitaxel is about 3,000 ng•h/mL to about 9,000 ng•h/mL.

In some embodiments, the AUC_{(0→∞)} of the orally administered paclitaxel is about 3,000 ng•h/mL to about 8,000 ng•h/mL.

In some embodiments, the AUC_{(0→∞)} of the orally administered paclitaxel is about 3,000 ng•h/mL to about 7,000 ng•h/mL.

In some embodiments, the AUC_{(0→∞)} of the orally administered paclitaxel is about 4,000 ng•h/mL to about 7,000 ng•h/mL.

In some embodiments, the AUC_{(0→∞)} of the orally administered paclitaxel is about 5,000 ng•h/mL to about 7,000 ng•h/mL.

In some embodiments, the AUC_{(0→∞)} of the orally administered paclitaxel is about 2,000 ng•h/mL.

In some embodiments, the AUC_{(0→∞)} of the orally administered paclitaxel is about 3,000 ng•h/mL.

In some embodiments, the AUC_{(0→∞)} of the orally administered paclitaxel is about 4,000 ng•h/mL.

In some embodiments, the AUC_{(0→∞)} of the orally administered paclitaxel is about 5,000 ng•h/mL.

In some embodiments, the AUC_{(0→∞)} of the orally administered paclitaxel is about 6,000 ng•h/mL.

In some embodiments, the AUC_{(0→∞)} of the orally administered paclitaxel is about 7,000 ng•h/mL.

In some embodiments, the AUC_{(0→∞)} of the orally administered paclitaxel is about 8,000 ng•h/mL.

In some embodiments, the AUC_{(0→∞)} of the orally administered paclitaxel is about 9,000 ng•h/mL.

In some embodiments, the AUC_{(0→∞)} of the orally administered paclitaxel is about 10,000 ng•h/mL.

The methods of the application include examples where the total amount of the paclitaxel orally administered per week is about 300 mg/m² to about 2,000 mg/m².

In some examples, the total amount of the paclitaxel orally administered per week is about 300 mg/m² to about 1,900 mg/m².

In some examples, the total amount of the paclitaxel orally administered per week is about 300 mg/m² to about 1,800 mg/m².

In some examples, the total amount of the paclitaxel orally administered per week is about 300 mg/m² to about 1,700 mg/m².

In some examples, the total amount of the paclitaxel orally administered per week is about 300 mg/m² to about 1,600 mg/m².

In some embodiments, the total amount of the paclitaxel orally administered per week is about 300 mg/m² to about 1,500 mg/m².

In some embodiments, the total amount of the paclitaxel orally administered per week is about 300 mg/m² to about 1,400 mg/m².

In some embodiments, the total amount of the paclitaxel orally administered per week is about 300 mg/m² to about 1,300 mg/m².

In some embodiments, the total amount of the paclitaxel orally administered per week is about 300 mg/m² to about 1,200 mg/m².

In some embodiments, the total amount of the paclitaxel orally administered per week is about 300 mg/m² to about 1,100 mg/m².

In some embodiments, the total amount of the paclitaxel orally administered per week is about 300 mg/m² to about 1,000 mg/m².

In some embodiments, the total amount of the paclitaxel orally administered per week is about 300 mg/m² to about 900 mg/m².

In some embodiments, the total amount of the paclitaxel orally administered per week is about 350 mg/m² to about 850 mg/m².

In some embodiments, the total amount of the paclitaxel orally administered per week is about 400 mg/m² to about 800 mg/m².

In some embodiments, the total amount of the paclitaxel orally administered per week is about 450 mg/m² to about 750 mg/m².

In some embodiments, the total amount of the paclitaxel orally administered per week is about 500 mg/m² to about 700 mg/m².

In some embodiments, the total amount of the paclitaxel orally administered per week is about 550 mg/m² to about 650 mg/m².

In some embodiments, the total amount of the paclitaxel orally administered per week is about 300, 325, 350, 375, 400, 425, 450, 475, 500, 525, 550, 575, 600, 605, 610, 615, 620, 625, 630, 635, 640, 645, 650, 655, 660, 665, 670, 675, 680, 685, 690, 695, 700, 725, 750, 775, 800, 825, 850, 875, 900, 925, 950, 975, 1,000, 1,025, 1,050, 1,075, 1,100, 1,125, 1,150, 1,175, 1,200, 1,225, 1,250, 1,275, 1,300, 1,325, 1,350, 1,375, 1,400, 1,425, 1,450, 1,475, or 1,500 mg/m². In some examples, the total amount of paclitaxel orally administered per week is about 1,525, 1,550, 1,575, 1,600, 1,625, 1,650, 1,675, 1,700, 1,725, 1,750, 1,775, or 1,800 mg/m².

In some examples, the total amount of the paclitaxel orally administered per week is at least about 300 mg/m² (*e.g*., at least 300 mg/m²).

In some embodiments, the total amount of the paclitaxel orally administered per week is at least about 350 mg/m² (*e.g*., at least 350 mg/m²).

In some embodiments, the total amount of the paclitaxel orally administered per week is at least about 400 mg/m² (*e.g.,* at least 400 mg/m²).

In some embodiments, the total amount of the paclitaxel orally administered per week is at least about 450 mg/m² (*e.g*., at least 450 mg/m²).

In some embodiments, the total amount of the paclitaxel orally administered per week is at least about 500 mg/m² (*e.g*., at least 500 mg/m²).

In some embodiments, the total amount of the paclitaxel orally administered per week is at least about 525 mg/m² *(e.g.,* at least 525 mg/m²).

In some embodiments, the total amount of the paclitaxel orally administered per week is at least about 550 mg/m² *(e.g.,* at least 550 mg/m²).

In some embodiments, the total amount of the paclitaxel orally administered per week is at least about 600 mg/m² *(e.g.,* at least 600 mg/m²).

In some embodiments, the total amount of the paclitaxel orally administered per week is at least about 615 mg/m² *(e.g.,* at least 615 mg/m²).

In some embodiments, the total amount of the paclitaxel orally administered per week is at least about 625 mg/m² *(e.g.,* at least 625 mg/m²).

In some embodiments, the total amount of the paclitaxel orally administered per week is at least about 630 mg/m² *(e.g.,* at least 630 mg/m²).

In some embodiments, the total amount of the paclitaxel orally administered per week is at least about 645 mg/m² *(e.g.,* at least 645 mg/m²).

In some embodiments, the total amount of the paclitaxel orally administered per week is at least about 650 mg/m² *(e.g.,* at least 650 mg/m²).

In some embodiments, the total amount of the paclitaxel orally administered per week is at least about 660 mg/m² *(e.g.,* at least 660 mg/m²).

In some embodiments, the total amount of the paclitaxel orally administered per week is at least about 675 mg/m² *(e.g.,* at least 675 mg/m²).

In some embodiments, the total amount of the paclitaxel orally administered per week is at least about 700 mg/m² *(e.g.,* at least 700 mg/m²).

In some embodiments, the total amount of the paclitaxel orally administered per week is at least about 750 mg/m² *(e.g.,* at least 750 mg/m²).

In some embodiments, the total amount of the paclitaxel orally administered per week is at least about 800 mg/m² *(e.g.,* at least 800 mg/m²).

In some embodiments, the total amount of the paclitaxel orally administered per week is at least about 825 mg/m² *(e.g.,* at least 825 mg/m²).

In some embodiments, the total amount of the paclitaxel orally administered per week is at least about 875 mg/m² *(e.g.,* at least 875 mg/m²).

In some embodiments, the total amount of the paclitaxel orally administered per week is at least about 900 mg/m² (*e.g.,* at least 900 mg/m²).

In some embodiments, the total amount of the paclitaxel orally administered per week is at least about 975 mg/m² (*e.g.,* at least 975 mg/m²).

In some embodiments, the total amount of the paclitaxel orally administered per week is at least about 1,000 mg/m² (*e.g.,* at least 1,000 mg/m²).

In some embodiments, the total amount of the paclitaxel orally administered per week is at least about 1,050 mg/m² (*e.g.,* at least 1,050 mg/m²).

In some embodiments, the total amount of the paclitaxel orally administered per week is at least about 1,100 mg/m² (*e.g.,* at least 1,100 mg/m²).

In some embodiments, the total amount of the paclitaxel orally administered per week is at least about 1,125 mg/m² (*e.g.,* at least 1,125 mg/m²).

In some embodiments, the total amount of the paclitaxel orally administered per week is at least about 1,200 mg/m² (*e.g.,* at least 1,200 mg/m²).

In some embodiments, the total amount of the paclitaxel orally administered per week is at least about 1,250 mg/m² (*e.g.,* at least 1,250 mg/m²).

In some embodiments, the total amount of the paclitaxel orally administered per week is at least about 1,300 mg/m² (*e.g.,* at least 1,300 mg/m²).

In some embodiments, the total amount of the paclitaxel orally administered per week is at least about 1,350 mg/m² (*e.g.,* at least 1,350 mg/m²).

In some embodiments, the total amount of the paclitaxel orally administered per week is at least about 1,375 mg/m² (*e.g.,* at least 1,375 mg/m²).

In some embodiments, the total amount of the paclitaxel orally administered per week is at least about 1,400 mg/m² (*e.g.,* at least 1,400 mg/m²).

In some embodiments, the total amount of the paclitaxel orally administered per week is at least about 1,500 mg/m² (*e.g.,* at least 1,500 mg/m²).

In some examples, the total amount of the paclitaxel orally administered per week is at least about 1,625 mg/m² (*e.g.,* at least 1,625 mg/m²).

In some examples, the total amount of the paclitaxel orally administered per week is at least about 1,650 mg/m² (*e.g.,* at least 1,650 mg/m²).

In some examples, the total amount of the paclitaxel orally administered per week is at least about 1,750 mg/m² (*e.g.,* at least 1,750 mg/m²).

In some examples, the total amount of the paclitaxel orally administered per week is at least about 1,800 mg/m² (*e.g.,* at least 1,800 mg/m²).

The methods of the application include examples where the AUC_{(0→∞)} of the orally administered paclitaxel is equal to or greater than the AUC_{(0→∞)} of intravenously administered paclitaxel (*e.g*., Taxol^{®} or paclitaxel formulated with Cremophor@) at an amount of about 80 mg/m² over a period of about 60 minutes in treating cancer.

The application describes to methods of treating cancer in a subject, and/or to methods for reducing or preventing toxicity, hypersensitivity-type infusion reactions and other negative outcomes resulting from or associated with intravenously administered paclitaxel (*e.g.*, Taxol^{®} or paclitaxel formulated with Cremophor@) therapy in a subject suffering from cancer.

The application pertains to a compound for use in the treatment of cancer in a subject, and/or to a compound for use in the reducing or preventing toxicity, hypersensitivity-type infusion reactions and other negative outcomes resulting from or associated with intravenously administered paclitaxel (*e.g*., Taxol^{®} or paclitaxel formulated with Cremophor@) therapy in a subject suffering from cancer.

The application describes to a use of a compound in the manufacture of a medicament for the treatment of cancer in a subject, and/or to a use of a compound in the manufacture of a medicament for the reducing or preventing toxicity, hypersensitivity-type infusion reactions and other negative outcomes resulting from or associated with intravenously administered paclitaxel (*e.g.,* Taxol^{®} or paclitaxel formulated with Cremophor@) therapy in a subject suffering from cancer.

The application describes to a use of a compound in a combination therapy for the treatment of cancer in a subject, and/or to a use of a compound in a combination therapy for the reducing or preventing toxicity, hypersensitivity-type infusion reactions and other negative outcomes resulting from or associated with intravenously administered paclitaxel (*e.g.*, Taxol^{®} or paclitaxel formulated with Cremophor@) therapy in a subject suffering from cancer.

The application describes to a use of a medicament in a combination therapy for the treatment of cancer in a subject, and/or to a use of a medicament in a combination therapy for the reducing or preventing toxicity, hypersensitivity-type infusion reactions and other negative outcomes resulting from or associated with intravenously administered paclitaxel (*e.g*., Taxol^{®} or paclitaxel formulated with Cremophor@) therapy in a subject suffering from cancer.

The embodiments of the application include examples where the intravenously administered paclitaxel is formulated in compositions that comprise paclitaxel and a pharmaceutical excipient or carrier that facilitates the intravenous administration of paclitaxel. In some embodiments, the intravenously administered paclitaxel is formulated with polyethoxylated castor oil, *e.g.,* Cremophor@. In some embodiments, the intravenously administered paclitaxel is Taxol^{®}, or a generic version thereof. In some embodiments, the intravenously administered paclitaxel is formulated with a protein carrier. In one embodiment, the intravenously administered paclitaxel is formulated in a composition comprising protein-bound paclitaxel, *i.e.,* Abraxane^{®}.

In some embodiments, the cancer is a disease that involves abnormal cell growth with the potential to invade or spread to other parts of the body.

In some embodiments, the cancer is a malignant tumor or neoplasm.

In a further embodiment, the cancer is breast cancer, pancreatic cancer, non-small cell lung cancer, ovarian cancer, AIDS-related Kaposi sarcoma, esophageal cancer, melanoma, lymphoma, uterine cancer, peritoneal cancer, fallopian tube cancer, endometrial cancer, cervical cancer, thyroid cancer, gastric cancer, gastroesophageal junction cancer, urothelial cancer, bladder cancer, oropharynx cancer, hypopharynx cancer, larynx cancer, head and neck cancer, germ cell cancer/tumors, prostate cancer, colon cancer, rectal cancer, kidney cancer, leukemia, or non-Hodgkin lymphoma.

In a further embodiment, the cancer is breast cancer, pancreatic cancer, non-small cell lung cancer, ovarian cancer, AIDS-related Kaposi sarcoma, esophageal cancer, melanoma, lymphoma, uterine cancer, peritoneal cancer, fallopian tube cancer, endometrial cancer, cervical cancer, thyroid cancer, gastric cancer, gastroesophageal junction cancer, urothelial cancer, bladder cancer, oropharynx cancer, hypopharynx cancer, larynx cancer, head and neck cancer, or germ cell cancer/tumors.

In a further embodiment, the cancer is breast cancer, non-small cell lung cancer, ovarian cancer, AIDS-related Kaposi sarcoma, esophageal cancer, bladder cancer, prostate cancer, or melanoma.

In one embodiment, the cancer is breast cancer, non-small cell lung cancer, ovarian cancer, or AIDS-related Kaposi sarcoma.

In one embodiment, the cancer is breast cancer. In a further embodiment, the breast cancer is metastatic breast cancer. In a further embodiment, the breast cancer is carcinoma of the breast.

In one embodiment, the cancer is lung cancer. In a further embodiment, the lung cancer is non-small cell lung cancer.

In one embodiment, the cancer is ovarian cancer. In a further embodiment, the cancer is carcinoma of the ovary.

In one embodiment, the cancer is AIDS-related Kaposi sarcoma.

In one embodiment, the cancer is pancreatic cancer. In a further embodiment, the pancreatic cancer is adenocarcinoma of the pancreas.

In one example, the disclosed methods include a method for treating metastatic breast cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 260 mg/m² over a period of about 30 minutes once every 3 weeks.

In one example, the disclosed methods include a method for treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 100 mg/m² over a period of about 30 minutes once per week for 3 weeks.

In one example, the disclosed methods include a method for treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² over a period of about 24 hours once every 3 weeks.

In one example, the disclosed methods include a method for treating adenocarcinoma of the pancreas in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 125 mg/m² over a period of about 30 minutes to about 40 minutes once per week for 3 weeks.

In one example, the disclosed methods include a method for treating carcinoma of the ovary in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to about 175 mg/m² over a period of about 3 hours once every 3 weeks.

In one example, the disclosed methods include a method for treating carcinoma of the breast in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 175 mg/m² once every 3 weeks.

In one example, the disclosed methods include a method for treating AIDS-related Kaposi's Sarcoma in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (e.g., Taxol^{®} or paclitaxel formulated with Cremophor@) at an amount of about 135 mg/m² over a period of about 3 hours once every 3 weeks, or at an amount of about 100 mg/m² over a period of about 3 hours once every 2 weeks.

In one embodiment, the application pertains to a compound for use in treating metastatic breast cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 260 mg/m² over a period of about 30 minutes once every 3 weeks.

In one embodiment, the application pertains to a compound for use in treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 100 mg/m² over a period of about 30 minutes once per week for 3 weeks.

In one embodiment, the application pertains to a compound for use in treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 135 mg/m² over a period of about 24 hours once every 3 weeks.

In one embodiment, the application pertains to a compound for use in treating adenocarcinoma of the pancreas in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 125 mg/m² over a period of about 30 minutes to about 40 minutes once per week for 3 weeks.

In one embodiment, the application pertains to a compound for use in treating carcinoma of the ovary in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to about 175 mg/m² over a period of about 3 hours once every 3 weeks.

In one embodiment, the application pertains to a compound for use in treating carcinoma of the breast in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 175 mg/m² once every 3 weeks.

In one embodiment, the application pertains to a compound for use in treating AIDS-related Kaposi's Sarcoma in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² over a period of about 3 hours once every 3 weeks, or at an amount of about 100 mg/m² over a period of about 3 hours once every 2 weeks.

In one example, the application describes a use of a compound in the manufacture of a medicament for treating metastatic breast cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 260 mg/m² over a period of about 30 minutes once every 3 weeks.

In one example, the application describes a use of a compound in the manufacture of a medicament for treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 100 mg/m² over a period of about 30 minutes once per week for 3 weeks.

In one example, the application describes a use of a compound in the manufacture of a medicament for treating non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² over a period of about 24 hours once every 3 weeks.

In one example, the application describes a use of a compound in the manufacture of a medicament for treating adenocarcinoma of the pancreas in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 125 mg/m² over a period of about 30 minutes to about 40 minutes once per week for 3 weeks.

In one example, the application describes a use of a compound in the manufacture of a medicament for treating carcinoma of the ovary in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to about 175 mg/m² over a period of about 3 hours once every 3 weeks.

In one example, the application describes a use of a compound in the manufacture of a medicament for treating carcinoma of the breast in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 175 mg/m² once every 3 weeks.

In one example, the application describes a use of a compound in the manufacture of a medicament for treating AIDS-related Kaposi's Sarcoma in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² over a period of about 3 hours once every 3 weeks, or at an amount of about 100 mg/m² over a period of about 3 hours once every 2 weeks.

In one embodiment, the application pertains to a compound for use in a combination therapy in the treatment of metastatic breast cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 260 mg/m² over a period of about 30 minutes once every 3 weeks.

In one embodiment, the application pertains to a compound for use in a combination therapy in the treatment of non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 100 mg/m² over a period of about 30 minutes once per week for 3 weeks.

In one embodiment, the application pertains to a compound for use in a combination therapy in the treatment of non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² over a period of about 24 hours once every 3 weeks.

In one embodiment, the application pertains to a compound for use in a combination therapy in the treatment of adenocarcinoma of the pancreas in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 125 mg/m² over a period of about 30 minutes to about 40 minutes once per week for 3 weeks.

In one embodiment, the application pertains to a compound for use in a combination therapy in the treatment of carcinoma of the ovary in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² to about 175 mg/m² over a period of about 3 hours once every 3 weeks.

In one embodiment, the application pertains to a compound for use in a combination therapy in the treatment of carcinoma of the breast in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 175 mg/m² once every 3 weeks.

In one embodiment, the application pertains to a compound for use in a combination therapy in the treatment of AIDS-related Kaposi's Sarcoma in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² over a period of about 3 hours once every 3 weeks, or at an amount of about 100 mg/m² over a period of about 3 hours once every 2 weeks.

In one embodiment, the application pertains to a medicament for use in a combination therapy in the treatment of metastatic breast cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 260 mg/m² over a period of about 30 minutes once every 3 weeks.

In one embodiment, the application pertains to a medicament for use in a combination therapy in the treatment of non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.*, Taxol^{®} or paclitaxel formulated with Cremophor@) at an amount of about 100 mg/m² over a period of about 30 minutes once per week for 3 weeks.

In one embodiment, the application pertains to a medicament for use in a combination therapy in the treatment of non-small cell lung cancer in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² over a period of about 24 hours once every 3 weeks.

In one embodiment, the application pertains to a medicament for use in a combination therapy in the treatment of adenocarcinoma of the pancreas in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 125 mg/m² over a period of about 30 minutes to about 40 minutes once per week for 3 weeks.

In one embodiment, the application pertains to a medicament for use in a combination therapy in the treatment of carcinoma of the ovary in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g*., Taxol^{®} or paclitaxel formulated with Cremophor@) at an amount of about 135 mg/m² to about 175 mg/m² over a period of about 3 hours once every 3 weeks.

In one embodiment, the application pertains to a medicament for use in a combination therapy in the treatment of carcinoma of the breast in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel *(*e.g., Taxol^{®} or paclitaxel formulated with Cremophor@) at an amount of about 175 mg/m² once every 3 weeks.

In one embodiment, the application pertains to a medicament for use in a combination therapy in the treatment of AIDS-related Kaposi's Sarcoma in a subject in need thereof, wherein the plasma exposure of the orally administered paclitaxel, as measured by AUC_{(0→∞)}, is equal to or greater than the plasma exposure, as measured by AUC_{(0→∞)}, of intravenously administered paclitaxel (*e.g.,* Taxol^{®} or paclitaxel formulated with Cremophor@) at an amount of about 135 mg/m² over a period of about 3 hours once every 3 weeks, or at an amount of about 100 mg/m² over a period of about 3 hours once every 2 weeks.

In one embodiment, the subject is fasted before paclitaxel and/or Compound A is orally administered. In one embodiment, the subject is fasted for at least 3 hours, at least 4 hours, at least 6 hours, at least 8 hours, at least 12 hours, at least 18 hours, or at least 24 hours. In one embodiment, the subject is fasted for at least 12 hours, at least 18 hours, or at least 24 hours before paclitaxel and/or Compound A is orally administered.

Unless explicitly indicated otherwise, the terms "approximately" and "about" are synonymous. In one embodiment, "approximately" and "about" refer to the recited amount, dose, value (for example, AUC_{(0→∞)}), or duration ± 20%, ± 15%, ± 10%, ± 8%, ± 6%, ± 5%, ± 4%, ± 2%, ± 1%, or ± 0.5%. In another embodiment, "approximately" and "about" refer to the listed amount, value, or duration ± 10%, ± 8%, ± 6%, ± 5%, ± 4%, or ± 2%. In yet another embodiment, "approximately" and "about" refer to the listed amount, value, or duration ± 5%. In yet another embodiment, "approximately" and "about" refer to the listed amount, value, or duration ± 2%. In yet another embodiment, "approximately" and "about" refer to the listed amount, value, or duration ± 1%. When the terms "approximately" and "about" are used when reciting temperature or temperature range, these terms refer to the recited temperature or temperature range ± 5 °C, ± 2 °C, or ± 1 °C. In another embodiment, the terms "approximately" and "about" refer to the recited temperature or temperature range ± 2 °C.

The term "paclitaxel" refers to 5,20-Epoxy-1,2,4,7,10-13-hexahydroxytax-11-en-9-one-4,10-diacetate 2-benzoate 13-ester with (2R,3S)-N-benzoyl-3-phenylisoserine, *i.e.,* the compound with the following structure: 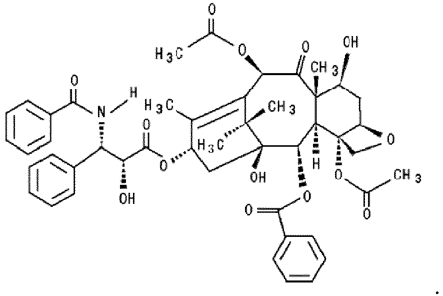 Unless otherwise indicated, the term "paclitaxel" includes pharmaceutically acceptable salts and/or solvates thereof.

Paclitaxel suitable for intravenous administration or intravenously administered paclitaxel includes compositions that comprise paclitaxel and a pharmaceutical excipient or carrier that facilitates the intravenous administration of paclitaxel. Such pharmaceutical excipient or carrier includes polyethoxylated castor oil, *e.g.,* Cremophor ^{®}. In one embodiment, paclitaxel suitable for intravenous administration or intravenously administered paclitaxel includes the brand name product, TAXOL^{®}, and generic versions thereof. In one embodiment, paclitaxel suitable for intravenous administration or intravenously administered paclitaxel includes a composition comprising protein-bound paclitaxel, *i.e.,* Abraxane^{®}.

Paclitaxel suitable for oral administration or orally administered paclitaxel refers to a formulation of paclitaxel that is administered orally. In one embodiment, the orally administered paclitaxel is in capsule form. In one embodiment, each capsule contains paclitaxel and a surfactant, *e.g*., polysorbate 80. In one embodiment, each capsule contains 30 mg - 200 mg of paclitaxel. In one embodiment, each capsule contains 30 mg, 45 mg, 60 mg, 75 mg, 90 mg, 100 mg, 125 mg, 150 mg, 175 mg, or 200 mg of paclitaxel. In one embodiment, each capsule contains 30 mg of paclitaxel. In one embodiment, each capsule contains 500 mg of polysorbate 80. In one embodiment each capsule contains 30 mg of paclitaxel and 500 mg of polysorbate 80. In one embodiment, the orally administered paclitaxel is in tablet form. In one embodiment, each tablet contains 30 mg - 200 mg of paclitaxel. In one embodiment, each tablet contains 30 mg, 45 mg, 60 mg, 75 mg, 90 mg, 100 mg, 125 mg, 150 mg, 175 mg, or 200 mg of paclitaxel. In one embodiment, each tablet contains 30 mg of paclitaxel.

Oral formulation of paclitaxel (*e.g.,* capsule or tablet) may be formulated by any suitable methods known in the art.

The term "Compound A" refers to a compound, or a pharmaceutically acceptable salt and/or solvate thereof, which is a P-gp pump inhibitor and has the following structure: 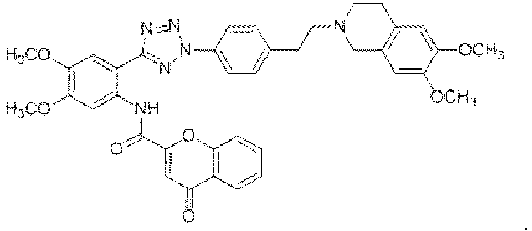 Unless indicated otherwise, the terms "Compound A," "HM30181 methanesulfonate monohydrate," "HM30181A," "HM30181AK," and "HM30181AK-US" are all equivalent and are used interchangeably. In one embodiment, Compound A refers to a methanesulfonate salt monohydrate of Compound A: 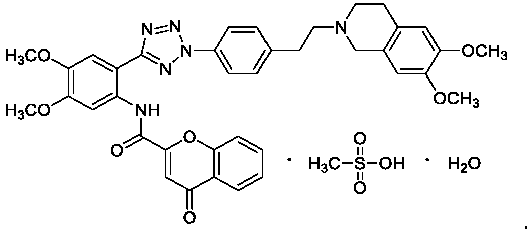 In one embodiment, Compound A refers to a methanesulfonate salt monohydrate of Compound A. Compound A is commercially available, *e.g.,* in tablet form suitable for oral administration. In one embodiment, Compound A is administered in 15 mg tablets suitable for oral administration.

Compound A may be formulated by any suitable methods known in the art.

The term "subject" includes any living organism that has cancer or is at a risk of developing cancer. In one embodiment, the term "subject" refers to a mammal that has cancer or is at a risk of developing cancer. In one embodiment, the term subject refers to a human being that has cancer or is at a risk of developing cancer. In one embodiment, the term subject refers to a cancer patient, *i.e*., a patient.

The term "AUC_{(0→∞)}" refers to the total exposure to a drug and is expressed in unit of concentration time. In one embodiment, it is the concentration of a drug over a time interval circulating in the body, *e.g.,* in the plasma, blood, or serum. In one embodiment, AUC_{(0→∞)} = AUC_{(0→Tlast)} + (C_{Tlast}/Kₑₗᵢₘ), where C_{Tlast} is the last measureable measurable drug concentration and Kₑₗᵢₘ is the terminal elimination rate constant, expressed in time⁻¹ units.

In one embodiment, C_{Tlast} may be determined from about 1 day to about 21 days after oral administration of paclitaxel.

In one embodiment, C_{Tlast} may be determined from about 2 days to about 14 days after oral administration of paclitaxel.

In one embodiment, C_{Tlast} may be determined from about 3 days to about 7 days after oral administration of paclitaxel.

In one embodiment, C_{Tlast} may be determined at about 3 days after oral administration of paclitaxel.

In one embodiment, C_{Tlast} may be determined at about 4 days after oral administration of paclitaxel.

In one embodiment, C_{Tlast} may be determined at about 5 days after oral administration of paclitaxel.

In one embodiment, C_{Tlast} may be determined at about 6 days after oral administration of paclitaxel.

In one embodiment, C_{Tlast} may be determined at about 7 days after oral administration of paclitaxel.

Hematologic toxicity associated with the intravenous administration of paclitaxel in a subject suffering from cancer can be assessed by a medical professional or health care worker by analyzing blood samples in a subject, *i.e*., determining cell counts, including white blood cells, absolute neutrophils, platelets, and hemoglobin.

Hypersensitivity-type infusion reactions, and symptoms associated with hematologic toxicity and/or neurotoxicity associated with the intravenous administration of paclitaxel in a subject suffering from cancer can be assessed by a medical professional or health care worker.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

The term "therapeutically effective amount", as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician.

For any compound, the therapeutically effective amount can be estimated initially either in cell culture assays or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, *e.g*., ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active ingredient or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

The pharmaceutical compositions containing paclitaxel may be manufactured in a manner that is generally known, *e.g.,* by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active ingredient into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating paclitaxel in the required amount in an appropriate solvent with one or a combination of ingredients enumerated above, as required, followed by filtered sterilization. Generally, dispersions are prepared by incorporating form paclitaxel into a sterile vehicle that contains a basic dispersion medium and the required other ingredients from those enumerated above. In the case of sterile powders for the preparation of sterile injectable solutions, methods of preparation are vacuum drying and freeze-drying that yields a powder of paclitaxel plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, form can be incorporated with excipients and used in the form of tablets, troches, or capsules. Oral compositions can also be prepared using a fluid carrier for use as a mouthwash, wherein in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

The pharmaceutical compositions described herein can be included in a container, pack, or dispenser together with instructions for administration.

All percentages and ratios used herein, unless otherwise indicated, are by weight.

Other features and advantages of the present application are apparent from the different examples. The provided examples illustrate different components and methodology useful in practicing the present application.

### EXAMPLES

### Example 1 - In vivo Anti-Cancer Activity in Tumor Cell Implant Mouse Models of Orally Administered Paclitaxel in combination with Compound A

In seven *in vivo* experiments in mice, the ability of orally administered paclitaxel in combination with Compound A to inhibit the growth of various human tumor transplant cell lines was studied. Paclitaxel alone, administered IV or intraperitoneally (IP), was used as control. Summary and results of the study are presented in the table below.

**Table 1**

| Mouse strain | Implant: human cancer cell line | Paclitaxel together with Compound A | | Paclitaxel Alone | |
|---|---|---|---|---|---|
| | | Paclitaxel Dose | Effect | Dose | Effect |
| NU/Nu Balb/C | SKOV-3 ovarian | 30 mg/kg 2x weekly for 3 weeks | 91.52 IR% | | |
| | | 40 mg 1x weekly for 3 weeks | 58.21 IR% | 30 mg/kg IV, 1x weekly for 3 weeks | 72.79 IR% |
| NU/Nu Balb/C | A431 vaginal epidermoid | 30 mg/kg 2x weekly for 3 weeks | 91.78 IR% | 30 mg/kg IV, 1x weekly for 3 weeks | 45.41 IR% |
| NU/Nu Balb/C | HT-29 colon | 50 mg/kg every 4 days for 4 doses | 96.28 IR% | 25 mg/kg IP every 4 days for 4 doses | 65.82 IR% |
| NU/Nu Balb/C | HT-29 colon | 40 mg/kg every 4 days for 4 doses | 40.21% tumor regression | 20 mg/kg IV every 4 days for 4 doses | 163.09% tumor regression |
| NCr-nu (athymic) | SK-OV-3 ovarian | 30 mg/kg every other day for 4 doses | >78.6 days growth delay | 18 mg/kg IV, every other day for 4 doses | 43.8 days growth delay |
| NCr-nu (athymic) | MDA-MB-231 mammary | 30 mg/kg every other day for 4 doses | >55.9 days growth delay | 18 mg/kg IV, every other day for 4 doses | >55.9 days growth delay |
| Athymic nude | NCI-H460 lung | 50mg/kg every other day for 4 doses | 12.7 days growth delay | 24 mg/kg IV, every other day for 4 doses | 16.3 days growth delay |

In these studies, tumor inhibition rate (IR%) was calculated as ([1-relative tumor growth in treated group/relative tumor growth in control group] × 100). Tumor regression was calculated as (Mean Tumor Weight_{End of treatment}/Mean tumor Weight_{Beginning of treatment}) ×100. Therefore, a value < 100% for tumor regression indicates a decrease in tumor weight over the course of the study and a value > 100% indicates an increase in tumor weight. Tumor growth delay was calculated as the median number of days to reach two tumor mass doublings.

In all studies, orally administered paclitaxel in combination with Compound A was active against transplanted human tumor cell lines. Paclitaxel administered IP or IV was generally less active. Because of differences in dose levels, comparative activity of the orally administered paclitaxel administered with IV or IP administered paclitaxel may not be reliably estimated in the absence of plasma or tissue exposure levels.

These results showed that oral administration of paclitaxel in combination with Compound A is effective in inhibiting the growth of various human tumor cell lines after implantation into mice. In addition, no adverse effects in a battery of CNS, respiratory and cardiovascular safety pharmacology studies were observed. Oral administration of paclitaxel in combination with Compound A caused minor reductions in body temperature in a CNS safety pharmacology study in rats, and reductions in PR and QRS intervals in a cardiovascular safety pharmacology in dogs. These effects are interpreted as being caused by paclitaxel, due to their absence when Compound A was administered alone.

### Example 2 - Dose Dependency of Orally Administered Paclitaxel

In rats and dogs administered with paclitaxel orally in combination with Compound A, Cₘₐₓ and AUC for paclitaxel increased in a greater than dose-proportional manner as shown in the tables below.

**Table 2: Dose-Dependency of Paclitaxel Pharmacokinetics in Rats After Single Dose Oral Administration in Combination with Compound A**

| **Dose Paclitaxel (mg/kg)** | **AUCₗₐₛₜ (ng.hr/mL)** | **Cₘₐₓ (ng/mL)** | **Tₘₐₓ (Hr)** | **t_{1/2} (Hr)** |
|---|---|---|---|---|
| | Paclitaxel | Paclitaxel | Paclitaxel | Paclitaxel |
| 5 | 333 | 103 | 0.6 | 8.2 |
| 10 | 1027 | 356 | 0.7 | 8.7 |
| 20 | 3009 | 852 | 1.2 | 7.5 |
| 30 | 6638 | 1761 | 1.2 | 6.0 |
| 40 | 11052 | 2488 | 1.5 | 5.5 |
| 50 | 13124 | 3067 | 1.4 | 5.3 |

**Table 3: Dose-Dependency of Paclitaxel Pharmacokinetics in Dogs After Single Dose Oral Administration in Combination with Compound A**

| **Dose Paclitaxel (mg/kg)** | **AUCₗₐₛₜ (ng.hr/mL)** | **Cₘₐₓ (ng/mL)** | **Tₘₐₓ (Hr)** | **t_{1/2} (Hr)** |
|---|---|---|---|---|
| | Paclitaxel | Paclitaxel | Paclitaxel | Paclitaxel |
| 2 | 413 | 128 | 0.5 | 7.4 |
| 4 | 1331 | 661 | 0.7 | 8.1 |
| 6 | 2123 | 1109 | 0.5 | 7.3 |

### Example 3 - Determination of the Bioavailability of Orally Administered Paclitaxel in Combination with Compound A in Subjects Suffering from Cancers

A study was designed to determine the absolute bioavailability of orally administered paclitaxel in combination with Compound A in subjects suffering from cancer. Eligible subjects were adults for whom therapy with intravenously administered paclitaxel (e.g., Taxol^{®} or paclitaxel formulated with Cremophor^{®}) at a dose of 80 mg/m² over 1 hour once per week is indicated.

The first 6 subjects received the following treatments approximately 1 week apart: 80 mg/m² intravenously administered paclitaxel (as Taxol^{®} or generic) infused over 1 hour (on day 1 or day 8) plus premedication per standard local practice; or 15 mg Compound A plus 270 mg (about 150 mg/m²) orally administered paclitaxel (days 1 and 2 or days 8 and 9). Compound A was administered 1 hour before oral paclitaxel. No premedication was allowed during this treatment sequence.

Based on preliminary results from the first 6 subjects, the protocol was amended to compare the extent of absorption of orally administered paclitaxel to that of 80 mg/m² intravenously administered paclitaxel (e.g., Taxol^{®} or paclitaxel formulated with Cremophor^{®}) at doses that were considered likely to meet criteria for bioequivalence by AUC_{(0→∞)}.

The amendment allowed for studying 2 additional subjects in a crossover design comparing oral paclitaxel at a daily dose of 274 mg/m² for 2 consecutive days and 80 mg/m² intravenously administered paclitaxel (e.g., Taxol^{®} or paclitaxel formulated with Cremophor^{®}) over 1 hour. Following the completion of the 6^{th} subject in the original 270 mg dose group, the protocol was further amended to compare the exposure of up to 2 additional cohorts of 2 subjects each at doses of orally administered paclitaxel that were increased by up to 25%, or decreased, if needed, to achieve a paclitaxel exposure similar to that of 80 mg/m² intravenously administered paclitaxel (e.g., Taxol^{®} or paclitaxel formulated with Cremophor^{®}) over 1 hour.

Based on preliminary data from 6 subjects at 270 mg per day for 2 days and 2 subjects at a daily dose of 274 mg/m² for 2 consecutive days, it was estimated that the bioavailability of orally administered paclitaxel is approximately 13.4%. Therefore, orally administered paclitaxel at a dose of 313 mg/m² daily for 2 consecutive days per week, *i.e.,* a weekly dose of 626 mg/m² orally administered paclitaxel, is expected to produce similar exposure to 80 mg/m² intravenously administered paclitaxel (*e.g*., Taxol^{®} or paclitaxel formulated with Cremophor^{®}) over 1 hour (within 80% to 125%).

### Example 4 - Determination of Maximum Tolerated Dose (MTD) for Orally Administered Paclitaxel in Combination with Compound A

To determine the MTD for orally administered paclitaxel, a Phase 1 study was conducted in 24 subjects with advanced solid cancer. An oral liquid formulation of paclitaxel was given on days 1, 8 and 15 in a cycle of 28 days. Paclitaxel doses ranged from 60 to 420 mg/m², and Compound A in tablet was dosed at half of paclitaxel doses (30 to 210 mg/m²) and administered 1 hour before paclitaxel.

**Table 4**

| Dose Level | Paclitaxel Dose (mg/m2) | Compound A Dose (mg/m2) |
|---|---|---|
| 1 | 60 | 30 |
| 2 | 120 | 60 |
| 3 | 180 | 90 |
| 4 | 240 | 120 |
| 5 | 300 | 150 |
| 6 | 360 | 180 |
| 7 | 420 | 210 |

The MTD was not reached in this study and dose escalation was stopped because of PK nonlinearity at paclitaxel doses above 300 mg/m². The results showed that paclitaxel Cₘₐₓ and AUC_{inf} increased with dose up to 300 mg/m² (Table 5). At doses above 300 mg/m², both Cₘₐₓ and AUC_{inf} plateaued. Clearance and elimination half-life of paclitaxel did not show significant change with increase in dose level. Mean apparent clearance (CL/F) ranged from 131.7 L/h to 178.4 at paclitaxel doses from 60 to 300 mg/m², but increased up to 244.9 L/h at higher doses. T_{1/2} ranged from 19.9-32.1 hours, consistent with published values for paclitaxel. Metabolic ratios of p-3-hydroxy paclitaxel and 6α-hydroxy paclitaxel metabolites were 0.1 - 0.25 and 0.04 - 0.13 respectively. Paclitaxel pharmacokinetics was linear and proportional with doses up to 300 mg/m². The overall toxicity profile of paclitaxel, when orally administered in combination with Compound A, was consistent with that of paclitaxel, with neutropenia being the adverse event associated with temporary or permanent discontinuation of treatment.

**Table 5: Pharmacokinetic Parameters of Paclitaxel by Dose Level**

| **Dose (mg/m2)** | **Statistic** | **tₘₐₓ^{a} (µg/L)** | **Cₘₐₓ (µg/L)** | **AUCₗₐₛₜ (µg^{∗}h/L)** | **AUC_{inf} (µg^{∗}h/L)** | **Vd/F (L)** | **CL/F (L/h)** | **t_{1/2} (h)** | **MRT_{inf} (h)** | **CL_{R} (L/h)** |
|---|---|---|---|---|---|---|---|---|---|---|
| **60 (n=2)** | **Mean** | 1.5 | 149.6 | 584.1 | 727.1 | 9128.7 | 178.4 | 32.1 | 28.6 | - |
| **120 (n=2)** | **Mean** | 1.0 | 655.3 | 1611.1 | 1840.1 | 4036.0 | 131.7 | 22.0 | 17.0 | 3.0 |
| **180 (n=2)** | **Mean** | 1.1 | 432.0 | 1673.9 | 1985.0 | 6030.7 | 173.4 | 24.4 | 21.9 | 1.8 |
| **240** | **Mean** | 1.0 | 509.6 | 2547.4 | 2971.2 | 6011.5 | 147.6 | 25.7 | 23.4 | 2.4 |
| **(n=3)** | | | | | | | | | | |
| | SD | 0.5 | 177.8 | 1000.3 | 941.1 | 4651.5 | 56.1 | 11.2 | 9.5 | 1.0 |
| | CV (%) | 1.0 | 34.9 | 39.3 | 31.7 | 77.4 | 38.0 | 43.6 | 40.7 | 39.4 |
| **300 (n=3)** | **Mean** | 0.5 | 919.7 | 3135.0 | 3481.1 | 4927.3 | 162.2 | 21.2 | 16.2 | 2.0 |
| | SD | 0.5 | 261.1 | 1450.1 | 1580.0 | 2900.7 | 98.4 | 1.4 | 0.5 | - |
| | CV (%) | 1.0 | 28.4 | 46.3 | 45.4 | 58.9 | 60.7 | 6.6 | 3.0 | - |
| **360 (n=3)** | **Mean** | 0.5 | 655.0 | 2512.1 | 2851.7 | 6837.8 | 228.7 | 20.8 | 18.7 | 7.3 |
| | SD | 0.5 | 464.8 | 809.9 | 923.1 | 1668.4 | 59.8 | 1.9 | 1.0 | 9.8 |
| | CV (%) | 2.0 | 71.0 | 32.2 | 32.4 | 24.4 | 26.1 | 9.0 | 5.5 | 134.3 |
| **420 (n=2)** | **Mean** | 0.5 | 707.5 | 3407.1 | 3731.5 | 7175.1 | 244.9 | 19.9 | 14.7 | 2.9 |

### Example 5 - Determination of MTD for Orally Administered Paclitaxel in Combination with Compound A

A study to determine the MTD for orally administered paclitaxel in subjects with advanced malignancies is being conducted. Successive cohorts are being treated with oral doses of 270 mg (approximately 150 mg/m²) paclitaxel per day. The initial cohort was treated with 270 mg per day 2 days per week for 3 weeks of a 4-week cycle. Subsequent cohorts received an additional day of treatment for each dosing week. Subjects in the first 2 cohorts (Arm 1) received 15 mg Compound A concomitantly with oral paclitaxel on Day 1 only, and were not required to fast prior to taking study medication. Subsequent cohorts (Arm 2) received Compound A concomitantly with each dose of paclitaxel and were explicitly instructed to fast prior to dosing. To date, the first 3 subjects have completed 5 days of dosing in Arm 2 (total dose 1350 mg or approximately 750 mg/m² paclitaxel per week for 3 out of 4 weeks).

### Example 6 - Clinical Study of Orally Administered Paclitaxel in Combination with Compound A

A 2-part study was conducted in subjects with advanced/metastatic or recurrent gastric cancer. The initial stage included a 28-day cycle MTD assessment of orally administered paclitaxel (as liquid filled capsules at doses of 90, 120, or 150 mg/m² per day for 2 days), with Compound A given concomitantly at a dose of 15 mg on Day 1. In the second part of this study, the selected dose of paclitaxel, 150 mg/m² per day, was given for 2 days per week, every 3 weeks out of a 4-week cycle. In some cases, an additional 15 mg tablet of Compound A on Day 2 was given in each dosing week. A total of 56 subjects enrolled in this study: 10 subjects with advanced malignant tumors in Part 1 and 46 subjects with advanced gastric cancer in Part 2.

Pharmacokinetic results from these 2 studies showed that paclitaxel maximum concentration (Cₘₐₓ) and area under the curve extrapolated to infinity (AUC_{inf}) increased with dose up to 300 mg/m² following administration of paclitaxel. At doses above 300 mg/m², both Cₘₐₓ and AUC_{inf} plateaued. Half-life (t_{1/2}) ranged from 19.9 to 32.1 hours, consistent with published values for paclitaxel. Metabolic ratios of p-3-hydroxy paclitaxel and 6α-hydroxy paclitaxel metabolites were 0.1 - 0.25 and 0.04 - 0.13, respectively. Following 2 consecutive daily doses of paclitaxel at doses of 60, 90, or 150 mg/m², minimal to no accumulation occurred in paclitaxel Cₘₐₓ and AUC. Cₘₐₓ and AUC₀₋₂₄ ranged from 202 - 280 (Day 1) and 159 - 315 ng/mL (Day 2), and 611 - 894 (Day 1) and 735 - 1081 ng•h/mL (Day 2), respectively, with minimal increase in Cₘₐₓ across the dose levels. Overall exposure on Day 2 was about 20 to 30% higher. T_{1/2} ranged from 18.3 to 19.7 hours, consistent with published values for paclitaxel. The results are shown below in Tables 6 and 7.

**Table 6: Paclitaxel Cₘₐₓ following Two Consecutive Daily Oral Doses**

| Dose (mg/m²) | Day | N | Mean (SD) (ng/mL) |
|---|---|---|---|
| 90 | 1 | 2 | 202 (111) |
| 120 | 1 | 3 | 280 (65.3) |
| 150 | 1 | 3 | 251 (100) |
| 90 | 2 | 2 | 315 (83.6) |
| 120 | 2 | 3 | 159 (114) |
| 150 | 2 | 3 | 276 (73.3) |

**Table 7: Paclitaxel AUC₀₋₂₄ Following Two Consecutive Daily Oral Doses**

| Dose (mg/m²) | Day | N | Mean (SD) (ng-hr/mL) |
|---|---|---|---|
| 90 | 1 | 2 | 611 (124) |
| 120 | 1 | 3 | 627 (207) |
| 150 | 1 | 3 | 894 (159) |
| 90 | 2 | 2 | 735 (171) |
| 120 | 2 | 3 | 904 (137) |
| 150 | 2 | 3 | 1081 (234) |

### Example 7: Food Effect

In dogs, absorption of orally administered paclitaxel was inhibited by food. Paclitaxel Cₘₐₓ and AUC were approximately a 2-fold higher in the fasted group as compared to the fed group.

**Table 8: Food Effect in Dogs after a Single Oral Dose of Paclitaxel in Combination with Compound A**

| | | **AUCₗₐₛₜ (ng.hr/mL)** | **Cₘₐₓ (ng/mL)** | **Tmax (hr)** | **t_{1/2} (hr)** | **Vd/F (L)** | **CL/F (L/hr)** | **AUC _{Fed}/ AUC_{Fasted} Ratio** |
|---|---|---|---|---|---|---|---|---|
| **Paclitaxel (6 mg/kg)** | Fed | 849.1± 119.9 | 299.0± 54.6 | 0.7±0.2 | 9.9±2.7 | 88.0± 19.6 | 6.4±1.3 | 53.1 |
| | Fasted | 1599.8± 416.5 | 683.6± 174.8 | 0.5±0.0 | 9.4±0.3 | 57.9± 17.4 | 4.2±1.2 | |

| | | | | | | | | |
|---|---|---|---|---|---|---|---|---|
| Dose: Paclitaxel, 6 mg/kg in combination with Compound A 3 mg/kg | | | | | | | | |

## Claims

1. A compound for use in the treatment of cancer in a subject in need thereof, wherein the subject is administered paclitaxel orally at an amount of about 100 mg/m² to about 250 mg/m² once a day and for 1-7 times a week;
wherein the compound is Compound A: 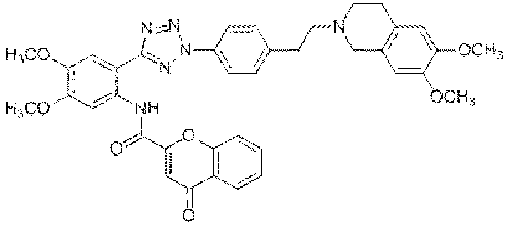 , which is administered to the subject at an amount of about 15 mg, about 20 mg, about 25 mg, or about 30 mg once a day and for 1-7 times a week; and
wherein Compound A is administered simultaneously with or prior to the paclitaxel.

2. A compound for use in reducing hematologic toxicity and/or neurotoxicity in a subject suffering from cancer and undergoing paclitaxel therapy, wherein the subject is administered paclitaxel orally at an amount of about 100 mg/m² to about 250 mg/m² once a day and for 1-7 times a week;
wherein the compound is Compound A: 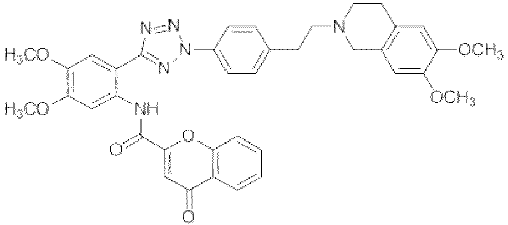 , which is administered to the subject at an amount of about 15 mg, about 20 mg, about 25 mg, or about 30 mg once a day and for 1-7 times a week; and
wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 135 mg/m² to 175 mg/m² over a period of about 3 to about 24 hours once every 3 weeks, and wherein Compound A is administered simultaneously with or prior to the paclitaxel.

3. The compound for use of claim 2, wherein the plasma exposure of the orally administered paclitaxel, as measured by AUC_{(0→∞)}, is equal to or greater than the plasma exposure, as measured by AUC_{(0→∞)}, of intravenously administered paclitaxel over a period of about 3 hours, or over a period of about 24 hours.

4. A compound for use in reducing or preventing hypersensitivity-type infusion reactions associated with intravenously administered paclitaxel therapy in a subject suffering from cancer, wherein the subject is administered paclitaxel orally at an amount of about 100 mg/m² to about 250 mg/m² once a day and for 1-7 times a week;
wherein the compound is Compound A: 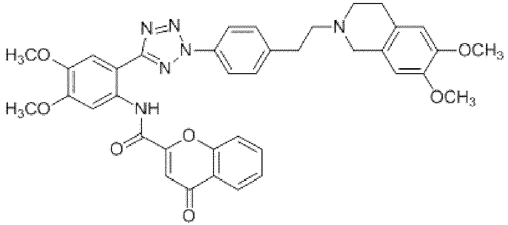
, which is administered to the subject at an amount of about 15 mg, about 20 mg, about 25 mg, or about 30 mg once a day and for 1-7 times a week; and
wherein the orally administered paclitaxel reaches therapeutic blood or plasma levels in the subject, and
wherein Compound A is administered simultaneously with or prior to the paclitaxel.

5. The compound for use of any one of the preceding claims, wherein the paclitaxel is administered at an amount of about 150 mg/m² to about 250 mg/m², about 150 mg/m², about 200 mg/m², about 250 mg/m², about 125 mg/m² to about 250 mg/m², or about 125 mg/m² to about 200 mg/m², about 100 mg/m² to about 250 mg/m², or about 100 mg/m² to about 200 mg/m².

6. The compound for use of any one of the preceding claims, wherein the paclitaxel is administered at least twice per week, 2-6 times per week, 2-5 times per week, at least 3 times per week, at least 4 times per week or 5 times per week.

7. The compound for use of any one of the preceding claims, wherein the plasma exposure of the orally administered paclitaxel, as measured by AUC_{(0→∞)}, is equal to or greater than the plasma exposure of intravenously administered paclitaxel at an amount of about 80 mg/m² over a period of about 60 minutes, as measured by AUC_{(0→∞)}, or wherein the AUC_{(0→∞)} of the orally administered paclitaxel is greater, at least about 10% greater, at least about 20% greater, at least about 30% greater, at least about 40% greater, at least about 50% greater, at least about 80% greater, or at least about 100% greater than the AUC_{(0→∞)} of intravenously administered paclitaxel at an amount of about 80 mg/m² over a period of about 60 minutes.

8. The compound for use of any one of the preceding claims, wherein the AUC_{(0→∞)} of the orally administered paclitaxel is about 2,000 ng•h/mL to about 10,000 ng•h/mL,
about 3,000 ng•h/mL to about 7,000 ng•h/mL or about 5,000 ng•h/mL to about 7,000 ng•h/mL.

9. The compound for use of any one of the preceding claims, wherein the total amount of the paclitaxel orally administered per week is about 300 mg/m² to about 1,500 mg/m².

10. The compound for use of any one of the preceding claims, wherein Compound A and the paclitaxel are administered on the same day or wherein Compound A is administered before the paclitaxel is administered.

11. The compound for use of any one of the preceding claims, wherein the AUC_{(0→∞)} of the orally administered paclitaxel is equal to or greater than the AUC_{(0→∞)} of intravenously administered paclitaxel at an amount of about 80 mg/m² over a period of about 60 minutes in treating cancer.

12. The compound for use of any one of the preceding claims, wherein the subject is suffering from metastatic breast cancer, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 260 mg/m² over a period of about 30 minutes once every 3 weeks, or
wherein the subject is suffering from non-small cell lung cancer, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 100 mg/m² over a period of about 30 minutes once per week for 3 weeks, or wherein the subject is suffering from adenocarcinoma of the pancreas, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 125 mg/m² over a period of about 30 minutes to about 40 minutes once per week for 3 weeks, or
wherein the subject is suffering from carcinoma of the ovary, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 135 mg/m² to about 175 mg/m² over a period of about 3 hours once every 3 weeks, or
wherein the subject is suffering from carcinoma of the breast, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 175 mg/m² once every 3 weeks, or
wherein the subject is suffering from non-small cell lung carcinoma, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 135 mg/m² over a period of about 24 hours once every 3 weeks, or wherein the subject is suffering from AIDS-related Kaposi's Sarcoma, and wherein the plasma exposure of the orally administered paclitaxel, as measured by AUC_{(0→∞)}, is equal to or greater than the plasma exposure, as measured by AUC_{(0→∞)}, of intravenously administered paclitaxel at an amount of about 135 mg/m² over a period of about 3 hours once every 3 weeks, or at an amount of about 100 mg/m² over a period of about 3 hours once every 2 weeks.

13. The compound for use of any one of the preceding claims, wherein the cancer is selected from the group consisting of breast cancer, pancreatic cancer, non-small cell lung cancer, ovarian cancer, AIDS-related Kaposi sarcoma, esophageal cancer, melanoma, lymphoma, uterine cancer, peritoneal cancer, fallopian tube cancer, endometrial cancer, cervical cancer, thyroid cancer, gastric cancer, gastroesophageal junction cancer, urothelial cancer, bladder cancer, oropharynx cancer, hypopharynx cancer, larynx cancer, head and neck cancer, germ cell cancer/tumors, prostate cancer, colon cancer, rectal cancer, kidney cancer, leukemia, and non-Hodgkin lymphoma,
preferably wherein the cancer is selected from the group consisting of breast cancer, pancreatic cancer, non-small cell lung cancer, ovarian cancer, AIDS-related Kaposi sarcoma, esophageal cancer, melanoma, lymphoma, uterine cancer, peritoneal cancer, fallopian tube cancer, endometrial cancer, cervical cancer, thyroid cancer, gastric cancer, gastroesophageal junction cancer, urothelial cancer, bladder cancer, oropharynx cancer, hypopharynx cancer, larynx cancer, head and neck cancer, and germ cell cancer/tumors,
more preferably wherein the cancer is selected from the group consisting of breast cancer, non-small cell lung cancer, ovarian cancer, AIDS-related Kaposi sarcoma, esophageal cancer, bladder cancer, prostate cancer, and melanoma,
most preferably wherein the cancer is selected from the group consisting of breast cancer, non-small cell lung cancer, ovarian cancer, and AIDS-related Kaposi sarcoma.

14. The compound for use of any one of the preceding claims, wherein the cancer is metastatic breast cancer.

15. The compound for use of any one of the preceding claims, wherein Compound A is administered orally at an amount of about 15 mg.

16. The compound for use of any one of the preceding claims, wherein the intravenously administered paclitaxel is paclitaxel in polyethoxylated castor oil or protein-bound paclitaxel.

## Patentansprüche

1. Verbindung zur Verwendung bei der Behandlung von Krebs bei einem Subjekt, das diese benötigt, wobei dem Subjekt Paclitaxel oral in einer Menge von etwa 100 mg/m² bis etwa 250 mg/m² einmal täglich und 1-7 mal pro Woche verabreicht wird;
wobei die Verbindung Verbindung A ist: 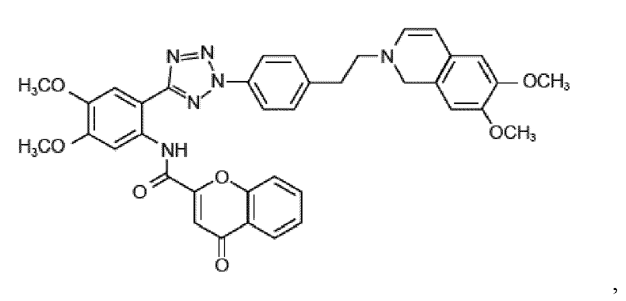 die dem Subjekt in einer Menge von etwa 15 mg, etwa 20 mg, etwa 25 mg oder etwa 30 mg einmal täglich und 1-7 mal pro Woche verabreicht wird; und
wobei die Verbindung A gleichzeitig mit oder vor dem Paclitaxel verabreicht wird.

2. Verbindung zur Verwendung beim Reduzieren von hämatologischer Toxizität und/oder Neurotoxizität bei einem dem Subjekt, das an Krebs leidet und einer Paclitaxel-Therapie unterzogen wird, wobei dem Subjekt Paclitaxel oral in einer Menge von etwa 100 mg/m² bis etwa 250 mg/m² einmal täglich und 1-7 mal pro Woche verabreicht wird;
wobei die Verbindung Verbindung A ist: 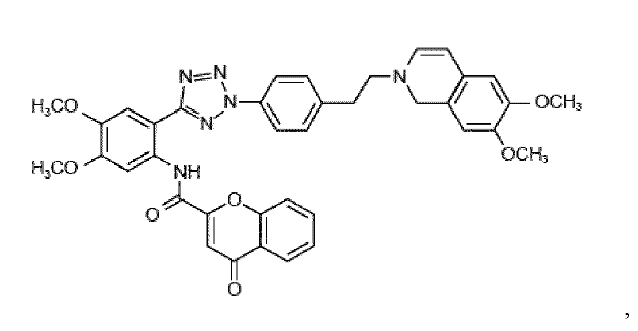 die dem Subjekt in einer Menge von etwa 15 mg, etwa 20 mg, etwa 25 mg oder etwa 30 mg einmal täglich und 1-7 mal pro Woche verabreicht wird; und
wobei die Plasmaexposition des oral verabreichten Paclitaxels, gemessen durch AUC_{(0→∞)}, gleich wie oder größer als die Plasmaexposition, gemessen durch AUC_{(0→∞)}, von intravenös verabreichtem Paclitaxel in einer Menge von etwa 135 mg/m² bis 175 mg/m² über einen Zeitraum von etwa 3 bis etwa 24 Stunden einmal alle 3 Wochen ist, und wobei Verbindung A gleichzeitig mit oder vor dem Paclitaxel verabreicht wird.

3. Verbindung zur Verwendung nach Anspruch 2, wobei die Plasmaexposition des oral verabreichten Paclitaxels, gemessen durch AUC_{(0→∞)} gleich wie oder größer als die Plasmaexposition, gemessen durch AUC_{(0→∞)}, von intravenös verabreichtem Paclitaxel über einen Zeitraum von etwa 3 Stunden oder über einen Zeitraum von etwa 24 Stunden ist.

4. Verbindung zur Verwendung beim Reduzieren oder Verhindern von Infusionsreaktionen vom Typ Hypersensibilität, die mit einer intravenös verabreichten Paclitaxel-Therapie bei einem an Krebs leidenden Subjekt assoziiert sind, wobei dem Subjekt Paclitaxel oral in einer Menge von etwa 100 mg/m² bis etwa 250 mg/m² einmal täglich und 1-7 mal pro Woche verabreicht wird;
wobei die Verbindung Verbindung A ist: 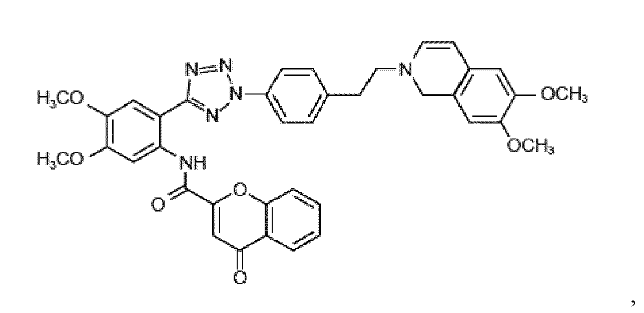 die dem Subjekt in einer Menge von etwa 15 mg, etwa 20 mg, etwa 25 mg oder etwa 30 mg einmal täglich und 1-7 mal pro Woche verabreicht wird; und
wobei das oral verabreichte Paclitaxel therapeutische Blut- oder Plasmaspiegel bei dem Subjekt erreicht, und
wobei die Verbindung A gleichzeitig mit oder vor dem Paclitaxel verabreicht wird.

5. Verbindung zur Verwendung nach einem der vorherigen Ansprüche, wobei das Paclitaxel in einer Menge von etwa 150 mg/m² bis etwa 250 mg/m², etwa 150 mg/m², etwa 200 mg/m², etwa 250 mg/m², etwa 125 mg/m² bis etwa 250 mg/m² oder etwa 125 mg/m² bis etwa 200 mg/m², etwa 100 mg/m² bis etwa 250 mg/m² oder etwa 100 mg/m² bis etwa 200 mg/m² verabreicht wird.

6. Verbindung zur Verwendung nach einem der vorherigen Ansprüche, wobei das Paclitaxel mindestens zweimal pro Woche, 2-6 mal pro Woche, 2-5 mal pro Woche, mindestens 3 mal pro Woche, mindestens 4 mal pro Woche oder 5 mal pro Woche verabreicht wird.

7. Verbindung zur Verwendung nach einem der vorherigen Ansprüche, wobei die Plasmaexposition des oral verabreichten Paclitaxels, gemessen durch AUC_{(0→∞)}, gleich wie oder größer als die Plasmaexposition von intravenös verabreichtem Paclitaxel in einer Menge von etwa 80 mg/m² über einen Zeitraum von etwa 60 Minuten, gemessen durch AUC_{(0→∞)} ist, oder wobei die AUC_{(0→∞)} des oral verabreichten Paclitaxels größer als mindestens etwa 10 % größer, mindestens etwa 20 % größer, mindestens etwa 30 % größer, mindestens etwa 40 % größer, mindestens etwa 50 % größer, mindestens etwa 80 % größer oder mindestens etwa 100 % größer ist als die AUC_{(0→∞)} von intravenös verabreichtem Paclitaxel in einer Menge von etwa 80 mg/m² über einen Zeitraum von etwa 60 Minuten.

8. Verbindung zur Verwendung nach einem der vorherigen Ansprüche, wobei die AUC_{(0→∞)} des oral verabreichten Paclitaxels etwa 2.000 ngeh/mL bis etwa 10.000 ngeh/mL, etwa 3.000 ngeh/mL bis etwa 7.000 ngeh/mL oder etwa 5.000 ngeh/mL bis etwa 7.000 ngeh/mL ist.

9. Verbindung zur Verwendung nach einem der vorherigen Ansprüche, wobei die Gesamtmenge des oral verabreichten Paclitaxels pro Woche etwa 300 mg/m² bis etwa 1.500 mg/m² ist.

10. Verbindung zur Verwendung nach einem der vorherigen Ansprüche, wobei Verbindung A und das Paclitaxel am selben Tag verabreicht werden oder wobei Verbindung A vor einer Verabreichung des Paclitaxels verabreicht wird.

11. Verbindung zur Verwendung nach einem der vorherigen Ansprüche, wobei die AUC_{(0→∞)} des oral verabreichten Paclitaxels gleich wie oder größer als die AUC_{(0→∞)}, von intravenös verabreichtem Paclitaxel in einer Menge von etwa 80 mg/m² über einen Zeitraum von etwa 60 Minuten beim Behandeln von Krebs ist.

12. Verbindung zur Verwendung nach einem der vorherigen Ansprüche, wobei das Subjekt an metastasierendem Brustkrebs leidet und wobei die Plasmaexposition des oral verabreichten Paclitaxels, gemessen durch AUC_{(0→∞)}, gleich wie oder größer als die Plasmaexposition, gemessen durch AUC_{(0→∞)}, von intravenös verabreichtem Paclitaxel in einer Menge von etwa 260 mg/m² über einen Zeitraum von etwa 30 Minuten einmal alle 3 Wochen ist, oder
wobei das Subjekt an nicht-kleinzelligem Lungenkrebs leidet und wobei die Plasmaexposition des oral verabreichten Paclitaxels, gemessen durch AUC_{(0→∞)} gleich wie oder größer als die Plasmaexposition, gemessen durch AUC_{(0→∞)} von intravenös verabreichtem Paclitaxel in einer Menge von etwa 100 mg/m² über einen Zeitraum von etwa 30 Minuten einmal pro Woche über 3 Wochen ist, oder wobei das Subjekt an einem Adenokarzinom der Bauchspeicheldrüse leidet und wobei die Plasmaexposition des oral verabreichten Paclitaxels, gemessen durch AUC_{(0→∞)} gleich wie oder größer als die Plasmaexposition, gemessen durch AUC_{(0→∞)}, von intravenös verabreichtem Paclitaxel in einer Menge von etwa 125 mg/m² über einen Zeitraum von etwa 30 Minuten bis etwa 40 Minuten einmal pro Woche über 3 Wochen ist, oder
wobei das Subjekt an einem Ovarialkarzinom leidet, und wobei die Plasmaexposition des oral verabreichten Paclitaxels, gemessen durch AUC_{(0→∞)}, gleich wie oder größer als die Plasmaexposition, gemessen durch AUC_{(0→∞)} von intravenös verabreichtem Paclitaxel in einer Menge von etwa 135 mg/m² bis etwa 175 mg/m² über einen Zeitraum von etwa 3 Stunden einmal alle 3 Wochen ist, oder
wobei das Subjekt an einem Karzinom der Brust leidet, und wobei die Plasmaexposition des oral verabreichten Paclitaxels, gemessen durch AUC_{(0→∞)} gleich wie oder größer als die Plasmaexposition, gemessen durch AUC_{(0→∞)} von intravenös verabreichtem Paclitaxel in einer Menge von etwa 175 mg/m² einmal alle 3 Wochen ist, oder
wobei das Subjekt an einem nicht-kleinzelligen Lungenkarzinom leidet und wobei die Plasmaexposition des oral verabreichten Paclitaxels, gemessen durch AUC_{(0→∞)} gleich wie oder größer als die Plasmaexposition, gemessen durch AUC_{(0→∞)}, von intravenös verabreichtem Paclitaxel in einer Menge von etwa 135 mg/m² über einen Zeitraum von etwa 24 Stunden einmal alle 3 Wochen ist, oder wobei
das Subjekt an AIDS-bedingtem Kaposi-Sarkom leidet, und wobei die Plasmaexposition des oral verabreichten Paclitaxels, gemessen durch AUC_{(0→∞)} gleich wie oder größer als die Plasmaexposition, gemessen durch AUC_{(0→∞)} von intravenös verabreichtem Paclitaxel in einer Menge von etwa 135 mg/m² über einen Zeitraum von etwa 3 Stunden einmal alle 3 Wochen oder in einer Menge von etwa 100 mg/m² über einen Zeitraum von etwa 3 Stunden einmal alle 2 Wochen ist.

13. Verbindung zur Verwendung nach einem der vorherigen Ansprüche, wobei der Krebs ausgewählt ist aus der Gruppe, bestehend aus Brustkrebs, Bauchspeicheldrüsenkrebs, nicht-kleinzelligem Lungenkrebs, Eierstockkrebs, AIDS-bedingtem Kaposi-Sarkom, Speiseröhrenkrebs, Melanom, Lymphom, Gebärmutterkrebs, Bauchfellkrebs, Eileiterkrebs, Endometriumkarzinom, Gebärmutterhalskrebs, Schilddrüsenkrebs, Magenkrebs, Krebs des gastroösophagealen Übergangs, Urothelkarzinom, Blasenkrebs, Oropharynxkarzinom, Hypopharynxkarzinom, Larynxkarzinom, Kopf- und Halskrebs, Keimzellenkrebs/-tumoren, Prostatakrebs, Dickdarmkrebs, Rektumkarzinom, Nierenkrebs, Leukämie und Nicht-Hodgkin-Lymphom,
vorzugsweise wobei der Krebs ausgewählt ist aus der Gruppe, bestehend aus Brustkrebs, Bauchspeicheldrüsenkrebs, nicht-kleinzelligem Lungenkrebs, Eierstockkrebs, AIDS-bedingtem Kaposi-Sarkom, Speiseröhrenkrebs, Melanom, Lymphom, Gebärmutterkrebs, Bauchfellkrebs, eileiterkrebs, Endometriumkrebs, Gebärmutterhalskrebs, Schilddrüsenkrebs, Magenkrebs, Krebs des gastroösophagealen Übergangs, Urothelkarzinom, Blasenkrebs, Oropharynxkarzinom, Hypopharynxkarzinom, Larynxkarzinom, Kopf- und Halskrebs und Keimzellenkrebs/-tumoren,
bevorzugter wobei der Krebs ausgewählt ist aus der Gruppe, bestehend aus Brustkrebs, nicht-kleinzelligem Lungenkrebs, Eierstockkrebs, AIDS-bedingtem Kaposi-Sarkom, Speiseröhrenkrebs, Blasenkrebs, Prostatakrebs und Melanom,
am meisten bevorzugt wobei der Krebs ausgewählt ist aus der Gruppe bestehend aus Brustkrebs, nicht-kleinzelligem Lungenkrebs, Eierstockkrebs und AIDS-bedingtem Kaposi-Sarkom.

14. Verbindung zur Verwendung nach einem der vorherigen Ansprüche, wobei der Krebs metastasierender Brustkrebs ist.

15. Verbindung zur Verwendung nach einem der vorherigen Ansprüche, wobei die Verbindung A oral in einer Menge von etwa 15 mg verabreicht wird.

16. Verbindung zur Verwendung nach einem der vorherigen Ansprüche, wobei das intravenös verabreichte Paclitaxel Paclitaxel in polyethoxyliertem Rizinusöl oder proteingebundenes Paclitaxel ist.

## Revendications

1. Composé destiné à être utilisé dans le traitement du cancer chez un sujet qui en a besoin, dans lequel le sujet reçoit du paclitaxel par voie orale en une quantité allant d'environ 100 mg/m² à environ 250 mg/m² une fois par jour et de 1 à 7 fois par semaine ;
dans lequel le composé est le composé A : 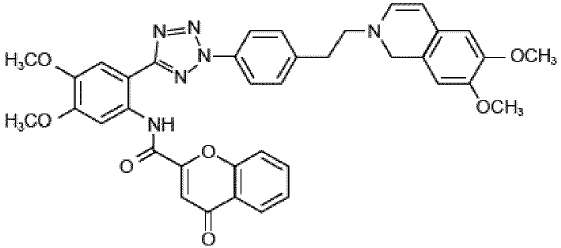 , qui est administré au sujet en une quantité d'environ 15 mg, d'environ 20 mg, d'environ 25 mg ou d'environ 30 mg une fois par jour et de 1 à 7 fois par semaine ; et
dans lequel le composé A est administré simultanément ou antérieurement au paclitaxel.

2. Composé destiné à être utilisé pour réduire la toxicité hématologique et/ou la neurotoxicité chez un sujet atteint d'un cancer et soumis à un traitement au paclitaxel, dans lequel le sujet reçoit du paclitaxel par voie orale en une quantité allant d'environ 100 mg/m² à environ 250 mg/m² une fois par jour et de 1 à 7 fois par semaine ;
dans lequel le composé est le composé A : 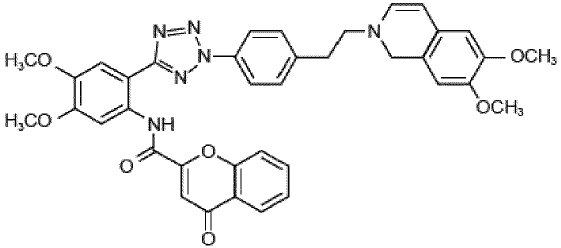 , qui est administré au sujet en une quantité d'environ 15 mg, d'environ 20 mg, d'environ 25 mg ou d'environ 30 mg une fois par jour et de 1 à 7 fois par semaine ; et
dans lequel l'exposition plasmatique du paclitaxel administré par voie orale, mesurée par AUC_{(0→∞)}, est supérieure ou égale à l'exposition plasmatique, mesurée par AUC_{(0→∞)}, du paclitaxel administré par voie intraveineuse en une quantité d'environ 135 mg/m² à 175 mg/m² sur une période allant d'environ 3 à environ 24 heures, une fois toutes les 3 semaines, et dans lequel le composé A est administré simultanément ou antérieurement au paclitaxel.

3. Composé destiné à être utilisé selon la revendication 2, dans lequel l'exposition plasmatique du paclitaxel administré par voie orale, mesurée par AUC_{(0→∞)}, est supérieure ou égale à l'exposition plasmatique, mesurée par AUC_{(0→∞)}, du paclitaxel administré par voie intraveineuse sur une période d'environ 3 heures, ou sur une période d'environ 24 heures.

4. Composé destiné à être utilisé pour réduire ou prévenir les réactions de perfusion de type hypersensibilité associées à un traitement par paclitaxel administré par voie intraveineuse chez un sujet souffrant d'un cancer, dans lequel le sujet reçoit du paclitaxel par voie orale en une quantité allant d'environ 100 mg/m² à environ 250 mg/m² une fois par jour et de 1 à 7 fois par semaine ;
dans lequel le composé est le composé A : 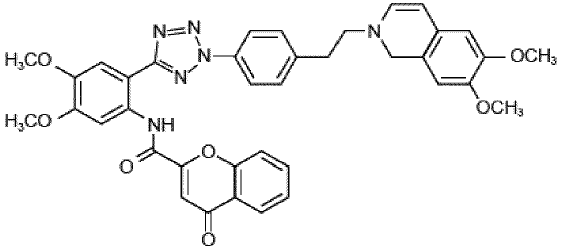 , qui est administré au sujet en une quantité d'environ 15 mg, d'environ 20 mg, d'environ 25 mg ou d'environ 30 mg une fois par jour et de 1 à 7 fois par semaine ; et
dans lequel le paclitaxel administré par voie orale atteint des niveaux sanguins ou plasmatiques thérapeutiques chez le sujet, et
dans lequel le composé A est administré simultanément ou antérieurement au paclitaxel.

5. Composé destiné à être utilisé selon l'une quelconque des revendications précédentes, dans lequel le paclitaxel est administré à une quantité allant d'environ 150 mg/m² à environ 250 mg/m², d'environ 150 mg/m², d'environ 200 mg/m², d'environ 250 mg/m², d'environ 125 mg/m² à environ 250 mg/m², ou d'environ 125 mg/m² à environ 200 mg/m², d'environ 100 mg/m² à environ 250 mg/m², ou d'environ 100 mg/m² à environ 200 mg/m².

6. Composé destiné à être utilisé selon l'une quelconque des revendications précédentes, dans lequel le paclitaxel est administré au moins deux fois par semaine, 2 à 6 fois par semaine, 2 à 5 fois par semaine, au moins 3 fois par semaine, au moins 4 fois par semaine ou 5 fois par semaine.

7. Composé destiné à être utilisé selon l'une quelconque des revendications précédentes, dans lequel l'exposition plasmatique du paclitaxel administré par voie orale, mesurée par AUC_{(0→∞)}, est supérieure ou égale à l'exposition plasmatique du paclitaxel administré par voie intraveineuse en une quantité d'environ 80 mg/m² sur une période d'environ 60 minutes, mesurée par AUC_{(0→∞)}, ou dans lequel l'AUC_{(0→∞)}, du paclitaxel administré par voie orale est supérieure, supérieure d'au moins environ 10 %, supérieure d'au moins environ 20 %, supérieure d'au moins environ 30 %, supérieure d'au moins environ 40 %, supérieure d'au moins environ 50 %, supérieure d'au moins environ 80 % ou supérieure d'au moins environ 100 % à l'AUC_{(0→∞)} du paclitaxel administré par voie intraveineuse en une quantité d'environ 80 mg/m² sur une période d'environ 60 minutes.

8. Composé destiné à être utilisé selon l'une quelconque des revendications précédentes, dans lequel l'AUC_{(0→∞)} du paclitaxel administré par voie orale se situe entre environ 2 000 ng•h/mL et environ 10 000 ng•h/mL, entre environ 3 000 ng•h/mL et environ 7 000 ng•h/mL ou entre environ 5 000 ng•h/mL et environ 7 000 ng•h/mL.

9. Composé destiné à être utilisé selon l'une quelconque des revendications précédentes, dans lequel la quantité totale de paclitaxel administrée par voie orale par semaine est d'environ 300 mg/m² à environ 1 500 mg/m².

10. Composé destiné à être utilisé selon l'une quelconque des revendications précédentes, dans lequel le composé A et le paclitaxel sont administrés le même jour ou dans lequel le composé A est administré antérieurement au paclitaxel.

11. Composé destiné à être utilisé selon l'une quelconque des revendications précédentes, dans lequel l'AUC_{(0→∞)}, du paclitaxel administré par voie orale est supérieure ou égale à l'AUC_{(0→∞)}, du paclitaxel administré par voie intraveineuse à une quantité d'environ 80 mg/m² sur une période d'environ 60 minutes dans le traitement du cancer.

12. Composé destiné à être utilisé selon l'une quelconque des revendications précédentes, dans lequel le sujet est atteint d'un cancer du sein métastatique, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 260 mg/m² sur une période d'environ 30 minutes, une fois toutes les 3 semaines, ou
dans lequel le sujet est atteint d'un cancer du poumon non à petites cellules, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 100 mg/m² sur une période d'environ 30 minutes, une fois par semaine pendant 3 semaines, ou dans lequel le sujet souffre d'un adénocarcinome du pancréas, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 125 mg/m² sur une période d'environ 30 minutes à environ 40 minutes, une fois par semaine pendant 3 semaines, ou
dans lequel le sujet souffre d'un carcinome de l'ovaire, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité allant d'environ 135 mg/m² à environ 175 mg/m² sur une période d'environ 3 heures, une fois toutes les 3 semaines, ou
dans lequel le sujet souffre d'un carcinome du sein, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 175 mg/m² une fois toutes les 3 semaines, ou
dans lequel le sujet souffre d'un carcinome pulmonaire non à petites cellules, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 135 mg/m² sur une période d'environ 24 heures, une fois toutes les 3 semaines, ou
dans lequel le sujet souffre d'un sarcome de Kaposi lié au SIDA, et dans lequel l'exposition plasmatique du paclitaxel administré par voie orale, mesurée par l'AUC_{(0→∞)}, est supérieure ou égale à l'exposition plasmatique, mesurée par l'AUC_{(0→∞)}, du paclitaxel administré par voie intraveineuse en une quantité d'environ 135 mg/m² sur une période d'environ 3 heures une fois toutes les 3 semaines, ou en une quantité d'environ 100 mg/m² sur une période d'environ 3 heures une fois toutes les 2 semaines.

13. Composé destiné à être utilisé selon l'une quelconque des revendications précédentes, dans lequel le cancer est choisi dans le groupe constitué par le cancer du sein, le cancer du pancréas, le cancer du poumon non à petites cellules, le cancer de l'ovaire, le sarcome de Kaposi lié au SIDA, le cancer de l'oesophage, le mélanome, le lymphome, le cancer de l'utérus, le cancer péritonéal, le cancer des trompes de Fallope, le cancer de l'endomètre, le cancer du col de l'utérus, le cancer de la thyroïde, le cancer de l'estomac, le cancer de la jonction gastro-oesophagienne, le cancer urothélial, le cancer de la vessie, le cancer de l'oropharynx, le cancer de l'hypopharynx, le cancer du larynx, le cancer de la tête et du cou, le cancer/les tumeurs des cellules germinales, le cancer de la prostate, le cancer du côlon, le cancer du rectum, le cancer du rein, la leucémie, et le lymphome non hodgkinien,
de préférence dans lequel le cancer est choisi dans le groupe constitué par le cancer du sein, le cancer du pancréas, le cancer du poumon non à petites cellules, le cancer de l'ovaire, le sarcome de Kaposi lié au SIDA, le cancer de l'oesophage, le mélanome, le lymphome, le cancer de l'utérus, le cancer du péritoine, le cancer des trompes de Fallope, le cancer de l'endomètre, le cancer du col de l'utérus, le cancer de la thyroïde, le cancer de l'estomac, le cancer de la jonction gastro-oesophagienne, le cancer urothélial, le cancer de la vessie, le cancer de l'oropharynx, le cancer de l'hypopharynx, le cancer du larynx, le cancer de la tête et du cou, le cancer/les tumeur des cellules germinales,
de préférence, le cancer est choisi dans le groupe constitué par le cancer du sein, le cancer du poumon non à petites cellules, le cancer de l'ovaire, le sarcome de Kaposi lié au SIDA, le cancer de l'oesophage, le cancer de la vessie, le cancer de la prostate et le mélanome,
le plus préférablement, le cancer est choisi dans le groupe constitué par le cancer du sein, le cancer du poumon non à petites cellules, le cancer de l'ovaire et le sarcome de Kaposi lié au SIDA.

14. Composé destiné à être utilisé selon l'une quelconque des revendications précédentes, dans lequel le cancer est un cancer du sein métastatique.

15. Composé destiné à être utilisé selon l'une quelconque des revendications précédentes, dans lequel le composé A est administré par voie orale en une quantité d'environ 15 mg.

16. Composé destiné à être utilisé selon l'une quelconque des revendications précédentes, dans lequel le paclitaxel administré par voie intraveineuse est du paclitaxel dans de l'huile de ricin polyéthoxylée ou du paclitaxel lié à des protéines.
